# EUROPEAN PATENT APPLICATION

(11) **EP 3 178 453 A1**
(43) Date of publication of application: **14.06.2017**
(21) Application number: 15828995.9
(22) Date of filing: 17.06.2015
(51) Int. Cl.: A61F 7/03

(54) **SELF-HEATING INSULATING FILM AND FACE MASK AND EYE MASK MANUFACTURED THEREFROM**

(30) Priority: 06.08.2014 CN 201410382367; 06.08.2014 CN 201410382376; 06.08.2014 CN 201410381994; 06.08.2014 CN 201410382368
(71) Applicant: Handy Technology (Zhuhai) Ltd., Zhuhai, Guangdong 519060 (CN)
(72) Inventor: HUANG, Yingluo, Zhuhai Guangdong 519099 (CN); CHEN, Lei, Zhuhai Guangdong 519099 (CN)
(74) Representative: Meyer, Thorsten
(86) International application number: PCT/CN2015/081597
(87) International publication number: WO 2016/019762

(57) **Abstract**

Disclosed is a self-heating thermal-insulation multilayer film comprising a structure of at least three layers, which from outside to inside are respectively: an outer layer (1) formed of an air permeable material; a heat-generating layer (2) loaded with heat-generating composition (21) that generates heat on contact with air, at least an upper sealing surface of the heat-generating layer that is in contact with the outer layer being formed of an air permeable material; and an thermal-insulation layer (3) formed of a material having waterproof and thermal-insulation properties. The present self-heating thermal-insulation film is structurally simple, is not constrained by environmental resources, does not require energy supply from an external energy source, is safe and stable, uniformly generates heat, and can effectively prolong heating time and reduce peak heating temperature. The present self-heating thermal-insulation film can be manufactured into a three-dimensional face mask, a face mask, or an eye mask for use on facial skin to improve blood circulation, enlarge facial pores, so as to clean skin dirt and sebaceous secretion. When used in combination with a traditional nourishing face mask or skincare product, the absorption of the effective ingredients in the traditional face mask or skincare product is improved, so the amount of skincare product used is reduced and skincare effect is enhanced. In addition, the use is safe and low-cost.

## Description

### FIELD OF THE INVENTION

The present invention relates to a self-heating thermal-insulation multilayer film, and particularly relates to a three-dimensional face mask, a face mask, or an eye mask made of the self-heating thermal-insulation multilayer film.

### BACKGROUND OF THE INVENTION

Now the self-heating pad, such as body warmer, utilizing the oxidation and self-heating mechanism has been widely used in commodity and healthcare markets to keep warm and relief cold dampness syndrome. The self-heating pad is generally made of a heat-generating composition, a package loading the heat-generating composition, an adhesive layer, an easily removeable covering layer adhering to the surface of the adhesive layer. When used, the covering layer is removed and the adhesive layer is adhered to the user's clothes, with the outer layer of the air-permeable package facing the skin, then the heat-generating composition generates heat as a result of coming into contact with water and oxygen in the air, so as to keep warm. However, all the existing self-heating pads have several shortcomings as follows: 1) the initial temperature is too high or the heat loss is too fast, so as to cause low temperature scalding and damage the body tissue, or result in a low holding temperature, that is, the heat distribution is disordered; 2) it is inhomogeneous in heating, so as to result in temperature of local part too high. In addition, these self-heating pads can't be used in combination with a skincare product, topically-applied medicine or healthcare product, since the skincare product, topically-applied medicine or healthcare product usually contains water or oil, the water or oil, together with the excreted sweat, would permeate into the outer layer of the air-permeable package facing the skin when the self-heating pads are in direct contact with the skin, so as to impact the chemical balance of the heat-generating composition and result in peak heating temperature uncontrollable. Furthermore, there is a risk of cross-contamination between the chemical reaction products of the heat- generating composition and the water or oil in the skincare product, or healthcare product or the sweat, so as to infect the skin.

A face mask is a carrier of cosmetics or skincare products, which is usually applied to the face for 15-30 minutes. When the cosmetics or skincare products have been absorbed by the skin, the face mask is taken off from the face. The most essential and important function of the face mask is to clean the skin, since it is not enough to clean the skin just by removing the make-up and washing the face. The face mask also has other functions, such as, skin moisturizing, skin whitening, or skin anti-aging.

Traditional face mask is usually a sheet-mask, which is covered on the face after unfolding. During the contact process, the sheet-mask can temporarily prevent outside air and pollutants from reaching the face, so as to increase the skin temperature, enlarge the skin pores, accelerate blood circulation and metabolism, and provide the skin with more oxygen, so that the metabolites produced by the epidermis cells and accumulated oils can be excluded from the skin. In addition, the water contained in the face mask can permeate into the stratum corneum of the skin, so as to soften and lighten the skin. The function of the traditional face mask is single, and the effect of cleaning skin and absorbing skincare products is limited.

The newly designed three-dimensional face mask includes a right side sheet and a left side sheet which are mutually symmetrical along the median line of the face, so that the three -dimensional face mask can be reliably applied to the user's face which has a concavo-convex shape. Compared with a sheet-mask, the three-dimensional face mask can improve the adherence over the user's face, and does not peel off easily. However, the function of the three -dimensional face mask is still single, and it does not improve the effect of cleaning skin and absorbing skincare products.

In order to make the nutritional ingredient in the face mask be absorbed by the skin, a routine method is to immerse the face mask in hot water or heat the face mask with vapor, so as to increase the temperature of the face mask, so that it is easier to enlarge the facial pores and clean skin dirt when applied to the face.

CN 20154766 A discloses a self-warming mask, which releases heat by the reaction of the exothermic composition in the thermal delivery pad. The heating temperature and holding time vary depending upon the permeability of the gas permeable polymer film, which, as the rate determining vehicle of the exothermic composition, controls the amount of oxygen exposed to the exothermic composition in the thermal delivery pad. However, the self -warming mask still fails to overcome the above shortcomings, such as, fast heat loss and uneven heating. Especially, when in combination with a skincare product or healthcare product, the water or oil in the skincare product or healthcare product, together with the excreted sweat, would permeate into the thermal delivery pad, so as to impact the chemical balance of the exothermic composition, and there is a risk of cross-contamination between the chemical reaction products of the exothermic composition and the water or oil in the skincare product, or healthcare product or the sweat, so as to infect the skin. In addition, the application fails to disclose that the effect of cleaning skin and absorbing skincare products can be enhanced by providing a face mask with an improved shape.

An eye mask, as a popular cosmetic product, is usually applied to the eyes, and performs an effective skin care around the eyes. The eye mask is capable of supplying the eyes with moisture and nutrition, relieving fatigue of eyes, eliminating dropsy and dark circles phenomenon. To some extent, the eye mask can also be used to reduce the eye wrinkle caused by aging.

With the popularity of electronic products in our daily life, people usually stare at the electronic screen for long hours, which strains the eyes and leads to eyes discomfort, fatigue or dry. When people lack sleep or have irregular sleep pattern, the damage to eyes is more serious. Therefore, eye care becomes more and more important.

Traditional eye mask is formed by mashing or boiling down the items which have eye care or cleaning function (such as, loofah, honey, tremella) and then applying the puree to the eyes. Commercial eye mask is a sheet-mask which is usually impregnated with an essence, such as, pentapeptide, sodium hyaluronate, vitamin E, vitamin B3, mineral substances or some natural ingredients, so as to achieve the effect of beauty and healthcare. However, the absorption effect of these eye masks is poor, since the natural permeation of the nutritional ingredient is limited.

For a hot compress, the eye mask is sometimes heated before using, such as, being immersed in hot water or treated with vapor, so as to relax the muscles around the eyes, dilate the blood vessels, or improve blood circulation. When the eye mask is heated before applying to the eyes, it is beneficial for the absorption of the nutritional ingredient, but the effect is still limited. In addition, due to the particularities of eyes, it is difficult to apply the above mentioned self-warming mask or paid to the eyes.

### SUMMARY OF THE INVENTION

The aim of the present invention is to provide a self-heating thermal-insulation multilayer film, throughout which the heat is distributed evenly, and which can effectively prolong heating time and reduce peak heating temperature, and can be used in combination with a skincare product, topically-applied medicine or healthcare product. The specific technical solution is as follows:

The self-heating thermal-insulation film comprises a structure of at least three layers, which from outside to inside are respectively: an outer layer formed of an air permeable material; a heat-generating layer loaded with heat-generating composition that generates heat on contact with air, at least an upper sealing surface of the heat-generating layer that is in contact with the outer layer being formed of an air permeable material; and an thermal-insulation layer formed of a material having waterproof and thermal-insulation properties. Compared with the existing products, the present self-heating thermal-insulation film comprises an additional thermal-insulation layer formed of a material having waterproof and thermal-insulation properties. When used, the thermal-insulation layer ensures the heat given off from the heat-generating layer is evenly and stably transferred to the skin, and the heat would not spread out quickly from the skin, so as to avoid scalding caused by too high initial temperature and maintain stable and suitable holding temperature. In addition, the thermal-insulation layer prevents the water or oil in the skincare product, topically-applied medicine or healthcare product or sweat from permeating into the heat-generating composition, so as to avoid uncontrollable peak heating temperature and cross-contamination between the chemical reaction products of the heat-generating composition and the water or oil in the skincare product, or healthcare product or the sweat, which may cause skin infection. The present self-heating thermal-insulation multilayer film is not limited to a structure of three layers, it may also comprise a package layer or adhesive layer etc. as needed.

Preferably, the thermal-insulation layer is made of metallized polyethylene terephthalate (MPET) film. In an example, the thermal-insulation layer is formed by a physical vapor deposition process, during which the PET film is coated with a layer of metal. The metal is heated and vaporized under vacuum, then the vaporized metal condenses or deposits on the cold PET film which is near the metal vapor source. The color of the MPET is usually golden or silvery. Of course, other colors may also be possible. In an example, aluminum is used for deposition or condensation, but other metals such as nickel or chromium or metal mixture can also be used. The MPET is much thinner than a metal foil, with a thickness equal to or less than 0.5µm. The MPET has good flexibility and waterproofness, and will not fade or discolor over time. Reflective golden or silvery surface can reflect heat back and prevent heat loss through radiation, so as to reserve more than 90% heat between the thermal-insulation layer and the skin. The addition of metal helps the heat given off from the heat-generating layer transfer to the skin evenly and stably, so as to increase and maintain skin temperature. In addition, golden or silvery surface can suit the user's aesthetic needs. Besides polyethylene terephthalate (PET), oriented polypropylene, nylon, polyethylene and cast polypropylene can also be used.

Preferably, the thermal-insulation layer is provided with griddings, and the heat-generating composition is evenly distributed in the griddings. In practice, the heat-generating composition is usually in powder or granule form. During use or transit, the powder or granule would accumulate in a certain area of the thermal-insulation layer due to gravity action, while there is no heat-generating composition in other areas. For example, when the film is applied to the face and the user is standing, the powdered or granular heat-generating composition will accumulate at the bottom of the thermal-insulation layer, so as to result in the bottom area too hot, while the upper area unwarming. The heat-generating composition can be evenly distributed in the griddings already set up, so that the thermal-insulation layer gives off heat evenly, so as to avoid scalding caused by local too high temperature. The specific number and shape of the griddings may vary depending upon the loaded heat-generating composition, the predetermined area of the film, for example the griddings may be oval, round, quadrate, floriated, pentagonal in shape, and the number may be 2 or more than 2.

Preferably, the griddings are formed by thermal bonding of the upper sealing surface of heat-generating layer in contact with the outer layer and the lower sealing surface of heat-generating layer in contact with the thermal-insulation layer. The griddings and the upper/lower sealing surface of heat-generating layer are directly molded in one. In production, the bonding mold of thermal-insulation layer is provided with gridding patterns. After heating, the upper/lower sealing surface will be bonded together on the bulge of the bolding mold. Of course, the griddings can be formed by any other known method in the prior art.

Preferably, the outer layer of the self-heating thermal-insulation film is made of nonwoven or woven fabric of natural or synthetic fiber. Nonwoven is usually made from polyester by a melt blown process. Nonwoven provides specific functions such as softness, air permeability, and its cost is rather low. In addition, nonwoven is similar to the air permeable material of the upper covering of heat-generating layer, so it is easier to composite the two materials.

In one embodiment, the self-heating thermal-insulation film is loaded in a sealed package before using, so as to prevent the heat-generating composition in heat-generating layer from being oxidized by oxygen and water in the air before using. The sealed package can be made from any air impermeable material, such as plastic film.

The heat-generating composition in heat-generating layer may contain oxidizable metal, active carbon, inorganic metal salt, water, polymer moisturizer, absorbent etc. The rationale of self-heating is that the oxidizable metal in the heat-generating layer can react with oxygen and water in the air (which is an exothermic chemical reaction), so as to generate heat and warm the skin. Other components in the heat-generating composition are used to accelerate the oxidation reaction.

The self-heating thermal-insulation film is ergonomically designed in shape to be applied to various body parts to be warmed, such as knee, back or wrist. There is no limitation to the shape of the self-heating thermal-insulation film, as long as it can match the body part to be applied to. The film can be adhered or attached to the user's body part by traditional chemical methods (such as, using an adhesive) or physical methods (such as, using a strap, velcro or fastener).

Preferably, in order to adhere the film to the user's body part, there is provided a layer of water-soluble hydrogel on the surface of the thermal-insulation layer that is in contact with the skin. Soluble hydrogel can be used as a body adhesive to adhere the film to the body part.

In soluble hydrogel, water is the dispersion medium. When soluble hydrogel is adhered to the skin, it turns from a solid to a liquid state due to body temperature, and permeates into the skin. Therefore, preferably, there is provided some active ingredients in the hydrogel matrix, such as, collagen, hyaluronic acid, arbutin, niacinamide, aromatic essential oil, or other healthcare products, topically-applied medicine etc. Since the present self-heating thermal-insulation film comprises an additional thermal-insulation layer formed of a material having waterproof and thermal-insulation properties, the water in the hydrogel can't permeate into the heat-generating layer, so as to avoid affecting peak heating temperature and heating effect. Furthermore, hydrogel is helpful to prevent heat from quickly spreading out from the skin.

In one embodiment, the self-heating thermal-insulation film is made into a mask applied to the user's face. Just as mentioned above, when the existing self-heating pad is used in combination with a skincare product or traditional nourishing mask, water and/or oil in the skincare product or nourishing mask, together with the excreted sweat, would permeate into the air-permeable layer facing the skin, so as to impact the chemical balance of the heat-generating composition and result in peak heating temperature uncontrollable. Furthermore, there is a risk of cross- contamination between the chemical reaction products of the heat-generating composition and the water or oil in the skincare product, or healthcare product or the sweat, so as to infect the skin. However, the present self-heating thermal-insulation film has overcome the above shortcomings, and has expanded the functionality (such as, skincare and self-heating function) of the traditional nourishing mask which only has single function.

Preferably, the heating temperature of the heat-generating layer is maintained at 38-55°C, and the heating time lasts 10-25 minutes. Within the above temperature range and time range, the face skin is comfortable, and blood circulation is accelerated, skin pores are enlarged, and sweat and accumulated oils are excluded from the skin. If the temperature is too low or the time is too short, skin pores can't be enlarged. If the temperature is too high or the time is too long, it may scald the epidermis of face skin in different degree, and the superficial capillary would expand quickly and/or keep in an expanding state for a long time, so as to cause moisture loss and rough pores. The heating temperature and the heating time can be controlled by adjusting the loading amount of the heat-generating composition, the ratio of ingredients in the heat-generating composition, the size and number of the aperture on the air-permeable outer layer or the upper sealing surface of the heat-generating layer. Generally speaking, the heat-generating layer is configured to heat the skin to an effective and adequate temperature, without causing any discomfort. For example, the self-heating thermal-insulation film can raise the temperature of the skin to 38-55°C, and the holding time depends upon the specific application, such as longer than 15minutes, 30minutes, or even 1 hour.

Preferably, the heat-generating composition in heat-generating layer comprises (by weigh): 30-50 parts medical iron powder; 10-15 parts active carbon; 1-5 parts metal salt; 3-13 parts vermiculite; 1-5 parts water-absorbent resin; and 1-5 parts water. In the case of conventional nonwoven or air-permeable material and conventional environmental condition, the designed formulation is capable of maintaining a temperature of 38-55°C for 10-25 minutes.

Preferably, the heat-generating composition in heat-generating layer comprises (by weigh): 30-50 parts medical iron powder; 10-15 parts active carbon; 1-5 parts metal salt; 3-13 parts vermiculite; 1-5 parts water-absorbent resin; 1-5 parts water; and 1-5 parts diatomite. Diatomite with ultramicropores can automatically absorb and store water in the air, so that excess water would not permeate into the heat-generating composition and cause too high temperature; and when the environment humidity is low, water stored in the ultramicropores of diatomite would be released, so as to prevent temperature from being too low due to inadequate water. The heating temperature of the present film is more stable by using diatomite to store or release water in the air.

Preferably, the mask is 2-8mm thick. Within such a thickness range, not only the structure of three layers and the above heating temperature and time can be achieved, but also the face mask fits tightly to the face. The face mask is routine in size.

The present invention also provides a method of using the self-heating thermal-insulation film. In some cases, the self-heating thermal-insulation film not only can be used to warm body, especially the body parts with rheumatism and paralysis, but also can be used to accelerate the absorption of topically-applied medicine or healthcare products. In one embodiment, a skincare product, topically-applied medicine, healthcare product or essential oil is applied to the body prior to applying the present film by a fixture. In one embodiment, a skincare product, topically-applied medicine, healthcare product or essential oil is pre-coated on the surface of thermal-insulation layer in contact with the skin by intrinsic viscidity, or added medical adhesive or thickener, and the pre-coated film is then directly applied to the skin. In addition, some essential oils or fragrant herbals can be added into the heat-generating composition, so that the present film can relax the user's body and mind while warming the body.

The present invention also provides a method of using the self-heating thermal-insulation face mask. A skincare product or traditional nourishing mark is applied to the face prior to applying the face mask. Since the present face mask can enlarge facial pores and accelerate the excretion of dirt and sebaceous secretion, the absorption of the effective ingredients in the skincare products is improved, the amount of skincare product used is reduced and skincare effect is enhanced.

The present self-heating thermal-insulation film is structurally simple, is not constrained by environmental resources, does not require energy supply from an external energy source, is safe and stable, uniformly generates heat, and can effectively prolong heating time and reduce peak heating temperature. Especially, when the self-heating thermal-insulation film is applied to the face as a face mask, the heat given off by the heat-generating layer and maintained by the thermal-insulation layer makes the skin comfortable, improves blood circulation and enlarges facial pores, so as to clean skin dirt and sebaceous secretion. When used in combination with a traditional nourishing face mask or skincare product, the absorption of the effective ingredients in the traditional face mask or skincare product is improved, so the amount of skincare product used is reduced and skincare effect is enhanced. In addition, the use is convenient, environmentally friendly and low-cost. The existing face mask only has single function, without self-heating function, so it is difficult for the skin to absorb the active ingredients (especially in the case that the environment temperature is low and the facial pores are constringed). In addition, during winter months or in cold areas, the cold feeling caused by traditional sheet-mask or mud-mask usually makes the user give up using the mask, so the skin is not nursed when the nutrient is needed the most. The present face mask has overcome the above shortcomings of the mask and has expanded the functionality of the existing mask.

Furthermore, the present self-heating thermal-insulation film is low cost and disposable, it is safe and hygienic when in contact with the face skin or other susceptible body parts, and avoids the need for disinfecting the physical heating apparatus which is reused and in direct contract with the skin and potential health risk caused by irregular disinfection. The used film will not cause toxin pollution, and the heat-generating composition can be recycled and used as materials improving the quality of the earth.

On the other hand, the present application provides a multifunctional three-dimensional face mask, throughout which the heat is distributed evenly, and which can effectively prolong heating time, reduce peak heating temperature, improve the absorption effect of skincare products, and prevent infection of face skin. The specific technical solution is as follows:

The face portion of the self-heating thermal-insulation three-dimensional face mask which has a facial concave-convex shape formed by joining two sheets together comprises a structure of at least three layers, which from outside to inside are respectively: an outer layer formed of air permeable material; a heat-generating layer loaded with heat-generating composition that generates heat on contact with air, at least an upper sealing surface of the heat-generating layer that is in contact with the outer layer being formed of an air permeable material; and an thermal-insulation layer formed of a material having waterproof and thermal-insulation properties. The present self-heating thermal-insulation three- dimensional face mask is not limited to a structure of the above three layers, it may also comprise a package layer or adhesive layer etc. as needed. In another aspect, the present film with a structure of the above three layers is in combination with a three-dimensional face mask which can fit the face better, so as to uniformly clean and nourish the face skin.

Preferably, the thermal-insulation layer of the three-dimensional face mask is made of metallized polyethylene terephthalate (MPET) film.

Preferably, the thermal-insulation layer of the three-dimensional face mask is provided with griddings, and the heat-generating composition is evenly distributed in the griddings.

Preferably, the griddings of the three-dimensional face mask are formed by thermal bonding of the upper sealing surface of heat-generating layer in contact with the outer layer and the lower sealing surface of heat-generating layer in contact with the thermal-insulation layer.

Preferably, the outer layer of the three-dimensional face mask is made of nonwoven or woven fabric of natural or synthetic fiber.

In one embodiment, the three-dimensional face mask is loaded in a sealed package before using.

The heat-generating composition in heat-generating layer of the three- dimensional face mask may contain oxidizable metal, active carbon, inorganic metal salt, water, polymer moisturizer, absorbent etc.

Preferably, the heating temperature of the heat-generating layer of the three-dimensional face mask is maintained at 38-55°C, and the heating time lasts 10-25 minutes.

Preferably, the heat-generating composition in heat-generating layer of the three-dimensional face mask comprises (by weigh): 30-50 parts medical iron powder; 10-15 parts active carbon; 1-5 parts metal salt; 3-13 parts vermiculite; 1-5 parts water-absorbent resin; and 1-5 parts water. In the case of conventional nonwoven or air-permeable material and conventional environmental condition, the designed formulation is capable of maintaining a temperature 38-55°C for 10-25 minutes.

Preferably, the heat-generating composition in heat-generating layer of the three-dimensional face mask comprises (by weigh): 30-50 parts medical iron powder; 10-15 parts active carbon; 1-5 parts metal salt; 3-13 parts vermiculite; 1-5 parts water-absorbent resin; 1-5 parts water; and 1-5 parts diatomite.

Preferably, the three-dimensional face mask is 2-8mm thick.

Preferably, in order to adhere the three-dimensional face mask to the user's face, there is provided a layer of water-soluble hydrogel on the surface of the thermal-insulation layer that is in contact with the skin.

In one embodiment, the three-dimensional face mask comprises a left side sheet and a right side sheet respectively matching the left area of the face and the right area of the face, front edge portions of the left side sheet and the right side sheet are provided with an adhesive material, the left side sheet and the right side sheet are thereby adhered together by the adhesive material along a median line of the face to form a facial concave-convex shape. Generally speaking, the left side sheet and the right side sheet are symmetrical along the median line of the face. Due to this, it is possible to keep this three-dimensional face mask completely adhering to every facial curve, so as to overcome the deficiencies of the traditional face mask, which is built from a single mask sheet and is liable to wrinkle and slack, thereby the traditional one-piece face mask cannot be uniformly and reliably applied to the face and affects the cosmetic and cleaning effect.

Preferably, either of the front edge portions of the left side sheet and the right side sheet has a protrusion projecting outwards from above down and matching the shape of the left area or right area of the external nose, a mouth portion which is a notch matching the mouth is provided below the protrusion, a portion of the front edge portion below the notch has an arc shape with a certain radian and extends downwards to the bottom the face mask; except the notch matching the mouth, either of the front edge portions of the left side sheet and the right side sheet is provided with an adhesive material, and the left side sheet and the right side sheet are thereby adhered together by the adhesive material. Because either of the front edge portions of the left side sheet and the right side sheet has a protrusion projecting outwards from above down and matching the shape of the left area or right area of the external nose, after the left side sheet and the right side sheet are adhered together, the three-dimensional face mask would naturally have a nose-like protrusion from above down on the user's nose, so as to reliably fit the nose. Because a mouth portion which is a notch matching the mouth is provided below the protrusion, and because a portion of the front edge portion below the notch has an arc shape with a certain radian and extends downwards to the bottom the face mask,, after the left side sheet and the right side sheet are adhered together, the lower half of face mask has a curved surface which can reliably fit the jaw, so as to improve the adherence to the face.

Preferably, the three-dimensional face mask according to the present invention is inclosed except that there is provided a hole corresponding to the user's mouth. Preferably, a glabella portion of either of the front edge portions of the left side sheet and the right side sheet which faces a space between the user's eyebrows, just above the protrusion, is formed as an arc which is slightly concaved inwards. The glabella portion is provided with an adhesive material. Due to this, after the left side sheet and the right side sheet are adhered together, the three-dimensional face mask would have a slight concave on a glabella-and- nasion portion, such that the three-dimensional face mask reliably fits the concave on the user's glabella-and-nasion portion. The three-dimensional face mask of this embodiment can clean and rejuvenate most areas of the face skin expect the mouth and nose. In addition, the heat-generating layer and thermal-insulation layer of the three-dimensional face mask can warm and nourish the user's eyes, and thus preventing dry eye syndrome.

Preferably, the three-dimensional face mask according to the present invention is provided with three holes respectively corresponding to the user's mouth and two eyes, that is, the user's mouth and eyes are not covered during use. An eye-and-glabella portion of either of the front edge portions of the left side sheet and the right side sheet which faces the user's eyes and glabella, just above his/her nose, is formed as a notch which is concaved inwards. The notch can be almost semielliptical or semicircular, as long as the eyes are uncovered but the skin around the eyes is almost covered. On either of the front edge portions of the left side sheet and the right side sheet, neither the mouth portion nor the eye-and-glabella portion is provided with adhesive material. That is, the left side sheet and the right side sheet are provided with an adhesive material on either of the front edge portions, except the portions corresponding to the user's mouth and eyes, and the left side sheet and the right side sheet are thereby adhered together by the adhesive material.

Preferably, there is a fixing strap provided on either of the rear edge portions of the left side sheet and the right side sheet, and there is a velcro provided at the end of the fixing strap. After applied to the face , the three-dimensional face mask can be attached to the user's head by the fixing strap and tighten by the velcro. In specific embodiments, the three-dimensional face mask can also be attached to the user's head by a lacing or fastener, not limited to a fixing strap and velcro.

The present invention also provides a method of using the self-heating thermal-insulation three-dimensional face mask. A skincare product or traditional nourishing mark is applied to the face prior to applying the three-dimensional face mask; or, a skincare product is pre-coated on the surface of thermal-insulation layer in contact with the skin, the left side sheet and the right side sheet are adhered together, then the pre-coated three-dimensional face mask is applied to the face. Since the present three-dimensional face mask can enlarge facial pores and accelerate the excretion of dirt and sebaceous secretion, the absorption of the effective ingredients in the skincare product is improved, the amount of skincare product used is reduced and skincare effect is enhanced. In addition, the thermal-insulation layer ensures the heat given off from the heat-generating layer is evenly and stably transferred to the skin, and the heat will not spread out quickly from the skin, so as to avoid scalding caused by too high initial temperature and maintain stable and suitable holding temperature. Furthermore, the thermal-insulation layer prevents the water or oil in the skincare product, topically-applied medicine or healthcare product or sweat from permeating into the heat-generating composition, so as to avoid uncontrollable peak heating temperature and cross-contamination between the chemical reaction products of the heat-generating composition and the water or oil in the skincare product, or healthcare product or the sweat, which may cause skin infection.

On the other hand, the present application provides a multifunctional self-heating thermal-insulation face mask, throughout which the heat is distributed evenly, and which can effectively prolong heating time, reduce peak heating temperature, improve the absorption effect of skincare products, and prevent infection of face skin. The specific technical solution is as follows:

The face portion of the self-heating thermal-insulation face mask comprises a structure of at least three layers, which from outside to inside are respectively: an outer layer formed of air permeable material; a heat-generating layer loaded with heat-generating composition that generates heat on contact with air, at least an upper sealing surface of the heat-generating layer that is in contact with the outer layer being formed of an air permeable material; and an thermal-insulation layer formed of a material having waterproof and thermal-insulation properties. The present self-heating thermal-insulation face mask is not limited to a structure of the above three layers, it may also comprise a package layer or adhesive layer etc. as needed.

Preferably, the thermal-insulation layer of the face mask is made of metallized polyethylene terephthalate (MPET) film.

Preferably, the thermal-insulation layer of the face mask is provided with griddings, and the heat-generating composition is evenly distributed in the griddings.

Preferably, the griddings of the face mask are formed by thermal bonding of the upper sealing surface of heat-generating layer in contact with the outer layer and the lower sealing surface of heat-generating layer in contact with the thermal-insulation layer.

Preferably, the outer layer of the face mask is made of nonwoven or woven fabric of natural or synthetic fiber.

In one embodiment, the face mask is loaded in a sealed package before using.

The heat-generating composition in heat-generating layer of the face mask may contain oxidizable metal, active carbon, inorganic metal salt, water, polymer moisturizer, absorbent etc.

Preferably, the heating temperature of the heat-generating layer the face mask is maintained at 38-55°C, and the heating time lasts 10-25 minutes.

Preferably, the heat-generating composition in heat-generating layer the comprises (by weigh): 30-50 parts medical iron powder; 10-15 parts active carbon; 1-5 parts metal salt; 3-13 parts vermiculite; 1-5 parts water-absorbent resin; and 1-5 parts water. In the case of conventional nonwoven or air-permeable material and conventional environmental condition, the designed formulation is capable of maintaining a temperature 38-55°C for 10-25 minutes.

Preferably, the heat-generating composition in heat-generating layer the face mask comprises (by weigh): 30-50 parts medical iron powder; 10-15 parts active carbon; 1-5 parts metal salt; 3-13 parts vermiculite; 1-5 parts water-absorbent resin; 1-5 parts water; and 1-5 parts diatomite.

Preferably, the face mask is 2-8mm thick.

Preferably, in order to adhere the face mask to the user's face, there is provided a layer of water-soluble hydrogel on the surface of the thermal-insulation layer that is in contact with the skin.

In one embodiment, the face mask has notches corresponding to the user's nose and mouth, respectively, so as to allow the user to breath during use of the face mask, and there is no notch provided on the portion corresponding to the user's eyes, so that the face mask can clean and rejuvenate most areas of the face expect the mouth and nose. In addition, the heat-generating layer and thermal-insulation layer of the face mask can warm and nourish the user's eyes, and prevent dry eye syndrome.

In another embodiment, besides the notches corresponding respectively to the user's nose and mouth, the face mask also has notches corresponding to the user's eyes, so that the user's sight and movement are not affected during use.

Further, there is a fixing strap provided on either side of the face mask, and there is a velcro provided at the end of the fixing strap. After applied to the face , the face mask can be attached to the user's head by the fixing strap and tighten by the velcro. In specific embodiments, the face mask can also be attached to the user's head by a lacing or fastener, not limited to a fixing strap and velcro.

The present invention also provides a method of using the self-heating thermal-insulation face mask. A skincare product or traditional nourishing mark is applied to the face prior to applying the present three-dimensional face mask; or, a skincare product is pre-coated on the surface of thermal-insulation layer in contact with the skin, then the face mask is applied to the face. Since the present face mask can enlarge facial pores and accelerate the excretion of dirt and sebaceous secretion, the absorption of the effective ingredients in the skincare product is improved, the amount of skincare product used is reduced and skincare effect is enhanced. In addition, the thermal-insulation layer ensures the heat given off from the heat-generating layer is evenly and stably transferred to the skin, and the heat will not spread out quickly from the skin, so as to avoid scalding caused by too high initial temperature and maintain stable and suitable holding temperature. Furthermore, the thermal-insulation layer prevents the water or oil in the skincare product, topically-applied medicine or healthcare product or sweat from permeating into the heat-generating composition, so as to avoid uncontrollable peak heating temperature and cross-contamination between the chemical reaction products of the heat-generating composition and the water or oil in the skincare product, or healthcare product or the sweat, which may cause skin infection.

On the other hand, the present application provides a multifunctional eye mask, throughout which the heat is distributed evenly, and which can effectively prolong heating time, reduce peak heating temperature, improve the absorption effect of skincare products, and prevent infection of eye skin. The specific technical solution is as follows:

The eye portion of the self-heating thermal-insulation eye mask comprises a structure of at least three layers, which from outside to inside are respectively: an outer layer formed of air permeable material; a heat-generating layer loaded with heat-generating composition that generates heat on contact with air, at least an upper sealing surface of the heat-generating layer that is in contact with the outer layer being formed of an air permeable material; and an thermal-insulation layer formed of a material having waterproof and thermal-insulation properties. The present self-heating thermal-insulation eye mask is not limited to a structure of the above three layers, it may also comprise a package layer or adhesive layer etc. as needed.

Preferably, the thermal-insulation layer of the eye mask is made of metallized polyethylene terephthalate (MPET) film.

Preferably, the thermal-insulation layer of the eye mask is provided with griddings, and the heat-generating composition is evenly distributed in the griddings.

Preferably, the griddings of the eye mask are formed by thermal bonding of the upper sealing surface of heat-generating layer in contact with the outer layer and the lower sealing surface of heat-generating layer in contact with the thermal-insulation layer.

Preferably, the outer layer of the eye mask is made of nonwoven or woven fabric of natural or synthetic fiber.

In one embodiment, the eye mask is loaded in a sealed package before using.

The heat-generating composition in heat-generating layer of the eye mask may contain oxidizable metal, active carbon, inorganic metal salt, water, polymer moisturizer, absorbent etc.

Preferably, the heating temperature of the heat-generating layer the eye mask is maintained at 38-55°C, and the heating time lasts 10-25 minutes.

Preferably, the heat-generating composition in heat-generating layer the comprises (by weigh): 30-50 parts medical iron powder; 10-15 parts active carbon; 1-5 parts metal salt; 3-13 parts vermiculite; 1-5 parts water-absorbent resin; and 1-5 parts water. In the case of conventional nonwoven or air-permeable layer, and conventional environmental condition, the designed formulation is capable of maintaining a temperature 38-55°C for 10-25 minutes.

Preferably, the heat-generating composition in heat-generating layer the eye mask comprises (by weigh): 30-50 parts medical iron powder; 10-15 parts active carbon; 1-5 parts metal salt; 3-13 parts vermiculite; 1-5 parts water-absorbent resin; 1-5 parts water; and 1-5 parts diatomite.

Preferably, the eye mask is 2-8mm thick.

Preferably, in order to adhere the eye mask to the user's eyes, there is provided a layer of water-soluble hydrogel on the surface of the thermal-insulation layer that is in contact with the skin.

Preferably, the eye mask comprises two hanging parts which have ears slits and can be hung on the user's ears. In use, the eye mask can be reliably attached to the user's eyes by the hanging parts, so the eye mask would not easily move or fall off from the eyes.

Preferably, a slit is vertically cut along a lower half of a central vertical axis of the eye mask. Due to this, after applied to the eyes, a left side piece and a right side piece of the eye mask can naturally splay outward on the portion of nose bridge, so as to fit the user's eyes in shape, that is, the left side piece and the right side piece of the eye mask are symmetrically arranged along the nose bridge and naturally splay outward to the two sides of the slit so as to be reliably adhered to the eyes.

A skincare product or traditional nourishing mark is applied to the skin around the eyes prior to applying the eye mask, a; or, a skincare product is pre-coated on the surface of thermal-insulation layer in contact with the skin, then the eye mask is applied to the eyes. Since the present eye mask can enlarge facial pores and accelerate the excretion of dirt and sebaceous secretion, the absorption of the effective ingredients in the skincare product is improved, the amount of skincare product used is reduced and skincare effect is enhanced. In addition, the thermal-insulation layer ensures the heat given off from the heat-generating layer is evenly and stably transferred to the skin, and the heat will not spread out quickly from the skin, so as to avoid scalding caused by too high initial temperature and maintain stable and suitable holding temperature. Furthermore, the thermal-insulation layer prevents the water or oil in the skincare product, topically-applied medicine or healthcare product or sweat from permeating into the heat-generating composition, so as to avoid uncontrollable peak heating temperature and cross-contamination between the chemical reaction products of the heat-generating composition and the water or oil in the skincare product, or healthcare product or the sweat, which may cause skin infection.

### BRIEF DESCRIPTION OF THE DRAWINGS

Figure 1 is a schematic view of a self-heating thermal-insulation multilayer film according to one embodiment of the present invention.
Figure 2 is a schematic view showing the self-heating thermal-insulation multilayer film of Figure 1, with the film being in use.
Figure 3 is a schematic view of a self-heating thermal-insulation multilayer film according to another embodiment of the present invention.
Figure 4 is a schematic view of a face mask made from the self-heating thermal-insulation multilayer film according to the present invention.
Figure 5 is a schematic showing the structure of the self-heating thermal-insulation three-dimensional face mask according to Example 4 of the present invention.
Figure 6 is a schematic view showing a face mask consisting of the right side sheet and left side sheet of Figure 5 when the right side sheet and left side sheet are adhered together.
Figure 7 is a schematic view showing a concave-convex shape of the three- dimensional face mask of Figure 5 when the three-dimensional face mask is attached to the user's face.
Figure 8 shows a sectional view of a face portion of the three-dimensional face mask of Figure 5, with the face portion having a structure of three layers.
Figure 9 is a schematic view showing the structure of a self-heating thermal-insulation three-dimensional face mask according to Example 5 of the present invention.
Figure 10 is a schematic view showing a face mask consisting of the right side sheet and left side sheet of Figure 9 when the right side sheet and left side sheet are adhered together.
Figure 11 is a schematic view showing a concave-convex shape of the three-dimensional face mask of Figure 9 when the three-dimensional face mask is attached to the user's face.
Figure 12 is a schematic view of a self-heating thermal-insulation face mask according to Example 8 of the present invention.
Figure 13 shows a sectional view of a face portion of the face mask of Figure 12, with the face portion having a structure of three layers.
Figure 14 is a schematic view of a self-heating thermal-insulation face mask according to Example 9 of the present invention.
Figure 15 is a schematic view of a self-heating thermal-insulation eye mask according to Example 12 of the present invention.
Figure 16 shows a sectional view of an eye portion of the eye mask of Figure 15, with the eye portion having a structure of three layers.
Figure 17 is a schematic view of a self-heating thermal-insulation eye mask according to Example 13 of the present invention.

### DETAILED DESCRIPTION OF ILLUSTRATED EMBODIMENTS

The present invention will be described in detail in connection with the following preferred embodiments, and it will be appreciated that such embodiments are merely exemplary, the invention is not to be limited to the disclosed embodiments, but on the contrary, is intended to cover various modifications and equivalent arrangements included within the spirit and scope thereof.

### Example 1

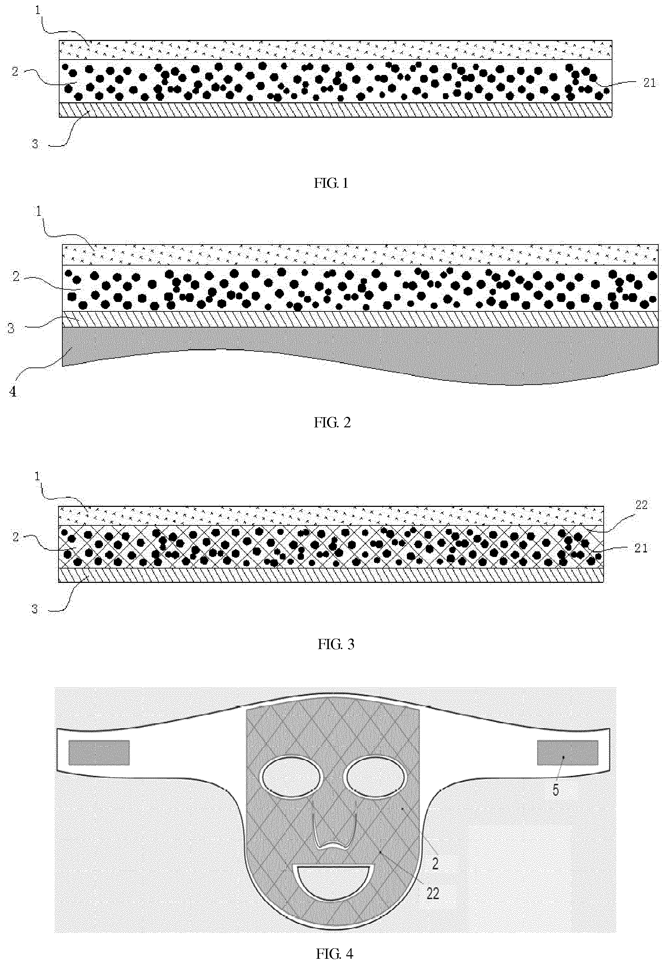

As shown in Figure 1, the self-heating thermal-insulation multilayer film comprises a structure of three layers: an outer layer 1, a heat-generating layer 2, and an thermal-insulation layer 3, and the heat-generating composition in powder or granule form is loaded in heat-generating layer 2. Figure 2 is a schematic view showing the state during use of the self-heating thermal-insulation multilayer film shown in Figure 1, the thermal-insulation layer 3 is in contact with the skin 4.

In this example, the above three layers of the self-heating thermal-insulation multilayer film are adhered together by, for example, the adhesive or thermal bonding, so they are applied to the skin as a whole. In some cases, the thermal-insulation layer is not adhered together with the outer layer and heat-generating layer. That is, in use, the thermal-insulation layer is first applied to the skin, then the outer layer and heat-generating layer are applied, and the similar self-heating thermal-insulation effect can also be obtained.

In this example, the outer layer 1 is made of nonwoven, and the upper sealing surface and lower sealing surface of the heat-generating layer 2 are made of air permeable film. In practice, as long as the outer layer and the upper sealing surface of the heat-generating layer (with or without the lower sealing surface) are made of air permeable material, there is no specific limitation to the air permeable material, all materials which can make air permeate into the heat-generating composition are suitable. The air permeability of the air permeable material can be controlled by adjusting the size and number of the aperture on the air-permeable film, which vary depending upon the heat-generating composition and the amount thereof, and the desired heating temperature. There is no limitation to the thickness of the air permeable material, as long as it has no affection on the desired effect. Thinner air permeable material is better while achieving the air-permeable property and supporting function. The thermal-insulation layer 3 is formed by a PET aluminum film made of PET and silvery aluminum. Aluminum has a lower melting point, so it can be easily changed from solid to gas by heating under vacuum. Compared with other film, PET film has better heat resistance and compatibility with aluminum. PET aluminum film has a reflective silvery surface, which can reflect heat back. The heat reflectivity of a PET aluminum film with a thickness of 400A°is almost 90%, so it has excellent heat-insulation effect. In addition, PET aluminum film also has good water and gas barrier property.

In this example, the heat-generating composition comprises (by weigh): 50 parts medical iron powder; 15 parts active carbon; 5 parts metal salt; 3 parts vermiculite; 1 part water-absorbent resin; 5 parts water; and 1 part diatomite. In practice, the heat-generating composition which can generate heat by reacting with oxygen in the air is a mixture of oxidizable metal, active carbon, inorganic metal salt, water, polymer moisturizer etc.

Examples of the oxidizable metals include iron and aluminum, iron (such as, reduced iron powder, atomized iron powder, electrolytic iron powder) is preferred. There is no limitation to the particle size of iron powder, as long as the likelihood and efficiency of the desired exothermal reaction is achieved, usually, 10 to 300µm, preferably 10 to 100µm. The iron powder is present in an amount of 30 to 80wt.%, preferably 30 to 50wt.%.

Active carbon may help dissipate heat energy equally, so as to avoid too high or too low local temperature. Active carbon has excellent water absorption capacity, which can also absorb the water vapor vaporized by the reaction heat, so as to prevent water from escaping, so active carbon can act as moisture retainers. In addition, active carbon can also absorb the bad smell produced during oxidization. The active carbon may be formed from coconut shells, wood, charcoal, coal, animal charcoal etc. There is no limitation to the particle size of active carbon as long as the likelihood and efficiency of the desired exothermal reaction is achieved, usually, 10 to 300µm, preferably 10 to 100µm. The active carbon is present in an amount of 3 to 25wt.%, preferably 10 to 15wt.%.

Inorganic metal salt makes it easier to perform oxidation of iron powder and oxygen. Inorganic metal salt can activate the surface of iron powder, so as to promote the oxidation of iron. Suitable inorganic metal salts may include sulfate, such as ferric sulfate, potassium sulfate, sodium sulfate, manganese sulfate, magnesium phosphate etc.; chloride, such as copper chloride, potassium chloride, sodium chloride, calcium chloride, manganese chloride, magnesium chloride, cuprous chloride etc. In addition, carbonate, nitrate, nitrate and other salts may also be used. These inorganic metal salts can be used independently or in combination. There is no limitation to the particle size of metal salts as long as the likelihood and efficiency of the desired exothermal reaction is achieved, usually, 10 to 700µm, preferably 200 to 600µm. The metal salt is present in an amount of 0.5 to 10wt.%, preferably 1 to 5wt.%.

Water contained in the heat-generating composition may be distilled water or tap water, which is present in an amount of 1 to 20wt.%, preferably 1 to 5wt.%.

Besides the above components, other additives may also be employed in the heat-generating composition as needed. For example, a water-absorbent resin may be employed, which may retain moisture in the heat-generating composition when the self-heating thermal-insulation multilayer film is encapsulated, furthermore, after the reaction has proceeded to a certain extent, the water-absorbent resin may release the moisture to allow the oxidation to continue. Suitable water-absorbent resins may include, but are not limited to, isobutylene/maleic acid copolymer, poly (vinyl alcohol)/acrylic copolymer, starch/acrylate graft copolymer, polyacrylate polymer, acrylate/acrylic acid copolymer, acrylate/ acrylamide copolymer, polyacrylonitrile etc. These water-absorbent resins can be used independently or in combination. There is no limitation to the particle size of water-absorbent resins as long as the likelihood and efficiency of the desired exothermal reaction is achieved, usually, 100 to 500µm, preferably 200 to 400µm. The water-absorbent resin is present in an amount of 0.5 to 10wt.%, preferably 1 to 5wt.%. The heat-generating composition may also contain vermiculite, which has thermal-insulation function, so as to maintain the temperature. There is no limitation to the particle size of vermiculite as long as the likelihood and efficiency of the desired exothermal reaction is achieved, usually, less than 300µ m ,preferably less than 200µm. The vermiculite is present in an amount of 1 to 20wt.%, preferably 3 to 13wt.%.

### Example 2

Figure 3 is a schematic view of a self-heating thermal-insulation multilayer film according to another embodiment. Compared with example 1, the heat-generating layer 2 in this example is provided with griddings 22, and the heat-generating composition in powder or granule form is evenly distributed in the griddings 22. Due to griddings 22, the heat-generating composition 21 in powder or granule form would not accumulate in a certain area of the thermal-insulation layer 2 under gravity action during use or transit, so that the thermal-insulation layer gives off heat evenly and local high temperature would not occur.

### Example 3

Figure 4 is a schematic view of a face mask made from the self-heating thermal-insulation multilayer film shown as Figure 3. Besides a structure of the above three layers and griddings 22, the face mask is provided with a fixture 5. The face mask is applied to the user's face, so it comprises no adhesive layer. A fixture 5 is provided to attach the face mask to the face. In practice, the fixture 5 may be a strap, velcro or fastener extending from the two sides of the face mask.

In this example, the heat-generating composition comprises (by weigh): 30 parts medical iron powder; 10 parts active carbon; 1 part metal salt; 13 parts vermiculite; 5 parts water-absorbent resin; 1 part water; and 1 part diatomite. The thickness of the face mask depends upon the heat-generating layer, since both the outer layer and thermal-insulation layer are made of films very thin. In order to maintain heating temperature of 38-55°Cand heating time of 10-25 minutes, the mask is 2-8mm thick, that is, the total heat-generating composition loaded in the heat-generating layer is 2-8mm thick. Within the thickness range, the mask fits tightly to the face.

### Technical advantage of Examples 1-3

In order to test the skincare and cosmetic effect of the present self-heating thermal-insulation multilayer film, 50 testees were recruited. All testees first applied a commercial available traditional mask of some brand to the whole face, then applied the self-heating thermal-insulation multilayer film in Example 3 to the left face or right face and held the film for 15 minutes. After removing the traditional mask and the present film, the testees were asked and observed how well the present film worked. As a result, these testees wildly believed that it was more comfortable on the side of the face applied with the present film, and it was verified by monitoring the changes of moisture content of the face skin that it had higher moisture content on the side of the face applied with both the present film and the traditional mask compared with the other side only applied with the traditional mask. In addition, it was observed that the side of the face applied with both the present film and traditional mask appeared more smooth and nutritious.

### Example 4

The example mainly relates to a three-dimensional face mask which has a facial concave-convex shape formed by joining two sheets together. Compared with the traditional face mask made from a single sheet of material, the three-dimensional face mask can fit the face better, so as to uniformly clean and nourish the face skin.

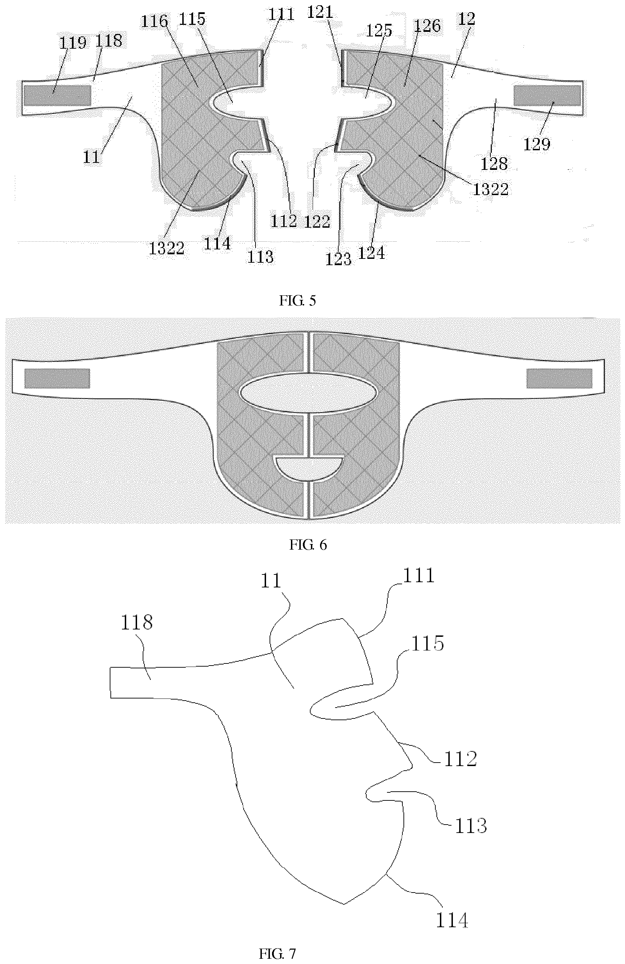

As shown in Figure 5, the three-dimensional face mask 10 comprises a left side sheet 11 and a right side sheet 12 respectively matching the left area of the face and the right area of the face. Either of the front edge portions 111, 121 of the left side sheet 11 and the right side sheet 12 is provided with an adhesive material, and the left side sheet 11 and the right side sheet 12 are thereby adhered together by adhesive material along the median line of the face. Figure 6 is a schematic view showing a face mask consisting of the right side sheet and left side sheet of Figure 5 when the right side sheet and left side sheet are adhered together.

In this example, the left side sheet 11 and the right side sheet 12 are symmetrical along the median line of the face. Of course, the present application is not limited to this, the left side sheet 11 and the right side sheet 12 may also be unsymmetrical in other examples. However, from the perspective of somatology, it is easier to fit the face when the two sheets are symmetrical. Since the two sheets are adhered together to form a facial concave-convex shape, it is possible to keep this three-dimensional face mask completely adhering to every facial curve, so as to overcome the deficiencies of the traditional face mask, which is built from a single mask sheet and is liable to wrinkle and slack, thereby the traditional one-piece face mask cannot be uniformly and reliably applied to the face and affects the cosmetic and cleaning effect.

As shown in Figure 5, either of the front edge portion 111 of the left side sheet 11 and the front edge portion 121 of the right side sheet 12 is provided with a protrusion 112,122 projecting outwards from above down and matching the shape of the left area or right area of the external nose. A notch 113,123 corresponding to the mouth is respectively provided below the protrusion112, 122. After the two sheets are joined together, the notch 113, 123 are joined and form a mouth portion matching the mouth. There is no adhesive material provided on the notch 113, 123 of the front edge portion 111,121. The portion of the front edge portion below the notch 113,123 respectively has an arc shape 114,124 with a certain radian and extends downwards to the bottom the face mask. Since either of the front edge portion 111 of the left side sheet 11 and front edge portion 121 of the right side sheet 12 has a protrusion projecting outwards from above down and matching the shape of the left area or right area of the external nose, after the left side sheet 11 and the right side sheet 12 are joined together, the three-dimensional face mask would naturally have a nose-like protrusion from above down on the user's nose, so as to reliably fit the nose. Since the portion of the front edge portion 111, 121 below the notch 113, 123 respectively has an arc shape with a certain radian and extends downwards to the bottom the face mask, after the left side sheet 11 and the right side sheet 12 are joined together, the lower half of the face mask has a curved surface which can reliably fit the jaw, so as to improve the adherence to the face.

Figure 7 shows the concave-convex shape of the three dimensional face mask which has been applied to the user's face. A nose-like protrusion is formed after the protrusion112, 122 are joined together, and an elliptical notch corresponding to the mouth is formed after the notch 113, 123 are joined together, and a curved surface is formed after the arc shape 114,124 are joined together, so as to fit the lower face and jaw. It can be seen that compared with the traditional sheet-mask, the present three-dimensional face mask fits the user's face which has a concave-convex shape better, so that it can be uniformly applied to the entire face and uniformly clean and nourish the face skin.

Preferably, a notch 114, 124 which is concaved inwards is provided on the eye-and-glabella portion of either of the front edge portion 111 , 121 of the left side sheet 11 and the right side sheet 12 which faces the user's eyes and glabella, just above the protrusion 112, 122. The notch 114, 124 can be almost semielliptical or semicircular, as long as the eyes are uncovered but the skin around the eyes is almost covered. On either of the front edge portions 111,121 of the left side sheet 11 and the right side sheet 12, neither the notch 113, 123 corresponding to the mouth nor the notch 114,124 corresponding to the eye-and-glabella is provided with adhesive material. That is, the left side sheet and the right side sheet are provided with an adhesive material on either of the front edge portions, except the notch 113, 123 corresponding to the mouth and the notch 114,124 corresponding to the eye-and-glabella, and the left side sheet and the right side sheet are thereby adhered together by the adhesive material.

Preferably, as shown in Figure 5, the rear edge portions of the left side sheet 11 and the right side sheet 12 are respectively provided with a fixing strap 118, 128, and preferably, the fixing strap 118, 128 are respectively provided with a velcro 119, 129. After applied to the face, the three-dimensional face mask can be attached to the user's head by the fixing strap and tighten by the velcro. The velcro, also known as hook-and-loop or hook-and-pile, is a common fastener used on the clothes, which is consisted of two components, typically, the first component features tiny hooks; the second features even smaller and "hairier" loops.

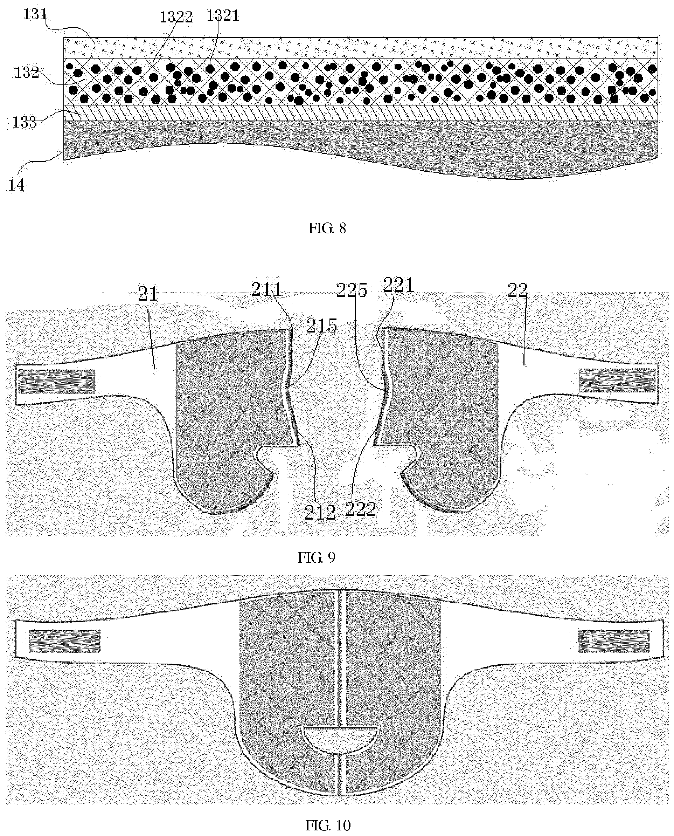

In this example, as shown in Figure 5, either of the face portions 116, 126 of the left side sheet 11 and the right side sheet 12 of the three-dimensional face mask 10 comprises a structure of at least three layers. Figure 8 shows the section view of the structure of three layers, which from outside to inside are respectively: an outer layer 131 formed of air permeable material; a heat-generating layer 132 loaded with heat-generating composition 1321 that generates heat on contact with air, at least an upper sealing surface of the heat-generating layer that is in contact with the outer layer being formed of an air permeable material; and an thermal-insulation layer 133 formed of a material having waterproof and thermal-insulation properties. The heat-generating composition 1321 in powder or granule form is loaded in heat-generating layer 132, and the thermal-insulation layer 133 is in contact with the skin 14.

In this example, the above three layers of the three-dimensional face mask are adhered together by, for example, using the adhesive or thermal bonding, which are applied to the skin as a whole. In some cases, the thermal-insulation layer is not adhered together with the outer layer and heat-generating layer. That is, in use, the thermal-insulation layer is first applied to the skin, then the outer layer and heat-generating layer are applied, and the similar self-heating thermal-insulation effect can also be obtained.

The face mask is mainly used for cleaning the skin. On the basis of cleaning, it also has other skincare effects, such as moisturizing, whitening and anti-aging. The aim of face mask improvement and development is to achieve these effects. In this example, the face portions of the two sheets comprises a structure of three layers, especially, an additional thermal-insulation layer formed of a material having waterproof and thermal-insulation properties is provided between the heat-generating layer and the skin. When used, the thermal-insulation layer ensures the heat given off from the heat-generating layer is evenly and stably transferred to the skin, and the heat will not spread out quickly from the skin, so as to avoid scalding caused by too high initial temperature and maintain stable and suitable holding temperature. In addition, the thermal-insulation layer prevents the water or oil in the skincare product, topically-applied medicine or healthcare product or sweat from permeating into the heat-generating composition, so as to avoid uncontrollable peak heating temperature and cross-contamination between the chemical reaction products of the heat-generating composition and the water or oil in the skincare product, or healthcare product or the sweat, which may cause skin infection. When applied to the face, the heat given off by the heat-generating layer and maintained by the thermal-insulation layer makes the skin comfortable, improves blood circulation and enlarges facial pores, so as to clean skin dirt and sebaceous secretion. When used in combination with a traditional nourishing face mask or skincare product, the absorption of the effective ingredients in the traditional face mask or skincare product is improved, so the amount of skincare product used is reduced and skincare effect is enhanced. In addition, the use is convenient, environmentally friendly and low-cost. Furthermore, besides the advantages brought by the above three layers, the present three-dimensional face mask can fit the face better, so as to uniformly clean and nourish the face skin. It can be said that the present three-dimensional face mask has made a real progress in cleaning and caring the skin in the field of face mask.

In this example, the outer layer 131 is made of nonwoven, the upper sealing surface and lower sealing surface of the heat-generating layer 132 are made of air permeable film, and the thermal-insulation layer 133 is a metal-plating film made of medicinal thermal-insulation film and silvery or golden metal. In practice, as long as the outer layer 131 and the upper sealing surface of the heat-generating layer 132 (with or without the lower sealing surface) are made of air permeable material, there is no specific limitation to the air permeable material, all materials which can make air permeate into the heat-generating composition are suitable. The air permeability of the air permeable material can be controlled by adjusting the size and number of the aperture on the air-permeable film, which vary depending upon the heat-generating composition and the amount thereof, and the desired heating temperature. There is no limitation to the thickness of the air permeable material, as long as it has no affection on the desired effect. Thinner air permeable material is better while achieving the air-permeable property and supporting function.

The thermal-insulation layer 133 is a metallized polyethylene terephthalate (MPET) film made of PET film and silvery or golden metal. In this example, the thermal-insulation layer is formed by a physical vapor deposition process, during which the PET film is coated with a layer of metal. The metal is heated and vaporized under vacuum, then the vaporized metal condenses or deposits on the cold PET film which is near the metal vapor source. In this example, aluminum is used for the MPET. Aluminum can be easily changed from solid to gas by heating under vacuum. Compared with other film, PET film has better heat resistance and compatibility with aluminum. The PET aluminum film has a reflective silvery surface, which can reflect heat back. The heat reflectivity of a PET aluminum film with a thickness of 400A°is almost 90%, so it has excellent heat- insulation effect. In addition, PET aluminum film also has good water and gas barrier property. In other example, other metals, such as nickel, chromium or metal mixture, can also be used. The MPET is much thinner than a metal foil, with a thickness equal to or less than 0.5µm. The MPET has good flexibility and waterproofness, and will not fade or discolor over time. Reflective golden or silvery surface can reflect heat back and prevent heat loss through radiation, so as to reserve more than 90% heat between the thermal-insulation layer and the skin. The addition of metal helps the heat given off from the heat-generating layer transfer to the skin evenly and stably, so as to increase and maintain skin temperature. In addition, golden or silvery surface can suit the user's aesthetic needs. Besides polyethylene terephthalate (PET), oriented polypropylene (OPP), nylon, polyethylene and cast polypropylene can also be used.

Further, the heat-generating layer 132 in this example is provided with griddings 1322, and the heat-generating composition in powder or granule form is loaded in each gridding 1322. Due to the griddings 1322, the heat-generating composition 1321 in powder or granule form would not accumulate in a certain area of the thermal-insulation layer 132 under gravity action during use or transit, so that the thermal-insulation layer gives off heat evenly and local high temperature would not occur.

In this example, the griddings 1322 are formed by thermal bonding of the upper sealing surface of heat-generating layer 132 in contact with the outer layer 131 and the lower sealing surface of heat-generating layer 132 in contact with the thermal-insulation layer 133. The griddings 1322 and the upper/lower sealing surface of heat-generating layer 132 are directly molded in one. In production, the bonding mold of thermal-insulation layer is provided with gridding patterns. After heating, the upper/lower sealing surface will be bonded together on the bulge of the bolding mold. Of course, the griddings can be formed by any other known method in the prior art.

In this example, the self-heating thermal-insulation three-dimensional face mask is loaded in a sealed package before using, so as to prevent the heat-generating composition in heat-generating layer from being oxidized by oxygen and water in the air before using. The sealed package can be made from any air impermeable material, such as plastic film.

In this example, the heat-generating composition comprises (by weigh): 50 parts medical iron powder; 15 parts active carbon; 5 parts metal salt; 3 parts vermiculite; 1 part water-absorbent resin; 5 parts water; and 1 part diatomite.

In this example, the thickness of the face mask depends upon the heat-generating layer, since both the outer layer and thermal-insulation layer are made of films very thin. In order to maintain heating temperature of 38-55°Cand heating time of 10-25 minutes, the mask is 2-8mm thick, that is, the total heat-generating composition loaded in the heat-generating layer is 2-8mm thick. Within the thickness range, the mask fits tightly to the face.

### Example 5

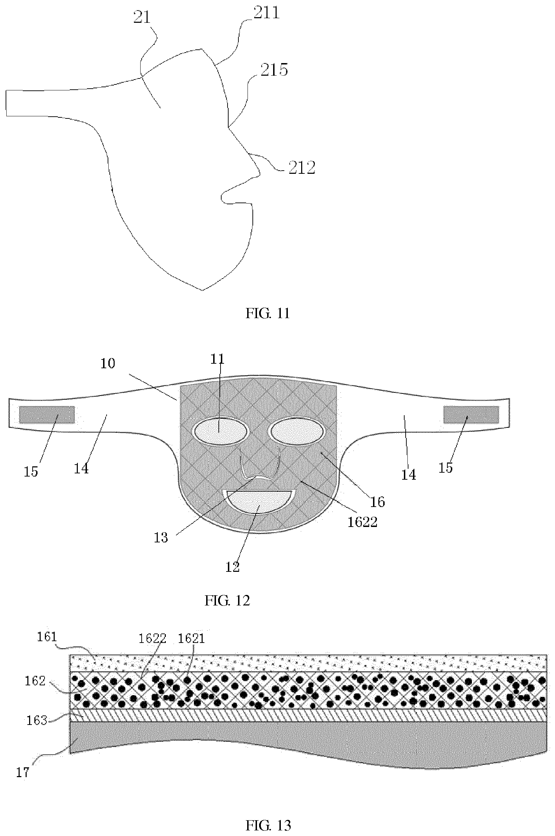

Figure 9 shows another example of the present three-dimensional face mask. The difference of example 5 over example 4 is that the three-dimensional face mask in example 5 is inclosed except that there is provided a notch corresponding to the user's mouth. That is to say, there is no notch provided on the eye and eyebrow portions of the front edge portion 211 of the left side sheet 21 and the front edge portion 221 of the right side sheet 22. In this example, either of the glabella portions of the front edge portions 211, 221 of the left side sheet 21 and the right side sheet 22 which faces a space between the user's eyebrows, just above the protrusion 212, 222, is formed as an arc 215, 225 which is slightly concaved inwards. The arc 215, 225 is provided with an adhesive material. Due to the arc 215, 225, after the left side sheet 21 and the right side sheet 22 are joined together, the three-dimensional face mask would have a slight concave on the arc 215, 225, such that the three-dimensional face mask reliably fits the concave on the user's glabella-and-nasion portion, just as shown in Figure 11.

The three-dimensional face mask in this example only has a notch on the portion corresponding to the mouth (including the portion below the nostril), other areas are inclosed, so it can clean and rejuvenate most areas of the face. In addition, the heat-generating layer and thermal-insulation layer of the three-dimensional face mask can warm and nourish the user's eyes, and thus preventing dry eye syndrome.

### Example 6

In this example, there is provided a layer of water-soluble hydrogel on the surface of the thermal-insulation layer that is in contact with the skin, so as to adhere the three-dimensional face mask to the user's face. Soluble hydrogel can be used as a body adhesive to adhere the film to the body.

In soluble hydrogel, water is the dispersion medium. When soluble hydrogel is adhered to the skin, it turns from a solid to a liquid state due to body temperature, and permeates into the skin. Therefore, preferably, there is provided some active ingredients in the hydrogel matrix, such as, collagen, hyaluronic acid, arbutin, niacinamide, aromatic essential oil, or other health care products, topically-applied medicine etc. Since the present self-heating thermal-insulation film comprises an additional thermal-insulation layer formed of a material having waterproof and thermal-insulation properties, the water in the hydrogel can't permeate into the heat-generating layer, so as to avoid affecting peak heating temperature and heating effect. Furthermore, hydrogel is helpful to prevent heat from quickly spreading out from the skin.

### Example 6

Example 7 differs from example 4 only in the heat-generating composition, which comprises (by weigh): 30 parts medical iron powder; 10 parts active carbon; 1 part metal salt; 13 parts vermiculite; 5 part water-absorbent resin; 1 part water; and 5 parts diatomite.

### Technical advantage of Examples 4-7

In order to test the skincare and cosmetic effect of the three-dimensional face mask, 50 testees were recruited. All testees first applied a commercial available traditional mask of some brand the whole face, then applied the three-dimensional face mask in Example 7 on their left face or right face and held the face mask for 15 minutes. After removing the traditional mask and the present three-dimensional face mask, the testees were asked and observed how well the three-dimensional face mask worked. As a result, these testees wildly believed that it was more comfortable on the side of the face applied with the present film, and it was verified by monitoring the changes of moisture content of the face skin that it had higher moisture content on the side of the face applied with both the present three-dimensional face mask and the traditional mask compared with the other side of the face only applied with the traditional mask. In addition, it was observed that the side of the face applied with both the present three-dimensional face mask and traditional mask appeared more smooth and nutritious.

The present three-dimensional face mask is structurally simple, is not constrained by environmental resources, does not require energy supply from an external energy source, is safe and stable, uniformly generates heat, and can effectively prolong heating time and reduce peak heating temperature. The heat given off by the heat-generating layer and maintained by the thermal-insulation layer makes the skin comfortable, improves blood circulation and enlarges facial pores, so as to clean skin dirt and sebaceous secretion. When used in combination with a traditional nourishing face mask or skincare product, the absorption of the effective ingredients in said traditional face mask or skincare product is improved, so the amount of skincare product used is reduced and skincare effect is enhanced. In addition, the use is convenient, environmentally friendly and low-cost. The existing face mask only has single function, without self-heating function, so it is difficult for the skin to absorb the active ingredients (especially in the case that the environment temperature is low and the facial pores are constringed). In addition, during winter months or in cold areas, the cold feeling caused by traditional sheet-mask or mud-mask usually makes the user give up using the mask, so the skin is not nursed when the nutrient is needed the most. The present three-dimensional face mask has overcome the above shortcomings of the existing mask and has expanded the functionality of the existing mask.

Furthermore, the present three-dimensional face mask is low cost and disposable, it is safe and hygienic when in contact with face skin or other susceptible body parts, and avoids the need for disinfecting the physical heating apparatus which is reused and in direct contract with the skin and potential health risk caused by irregular disinfection. The used face mask will not cause toxin pollution, and the heat-generating composition can be recycled and used as materials improving the quality of the earth.

### Example 8

As shown in Figure 12, in example 8, the face mask 10 has notches 11 corresponding to the user's eyes, and notch 12 corresponding to the mouth, and notch 13 corresponding to the nostril. The two sides of the face mask 10 are provided with a fixing strap 14, and the fixing strap 14 is provided with a velcro 15.

After applied to the face, the face mask 10 can be attached to the user's head by the fixing strap 14 and tighten by the velcro 15.

In this example, as shown in Figure 12, the face portion 16 of the face mask 10 comprises a structure of at least three layers. Figure 13 shows the section view of the structure of three layers, which from outside to inside are respectively: an outer layer 161 formed of air permeable material; a heat-generating layer 162 loaded with heat-generating composition 1621 that generates heat on contact with air, at least an upper sealing surface of the heat-generating layer that is in contact with the outer layer being formed of an air permeable material; and an thermal-insulation layer 163 formed of a material having waterproof and thermal-insulation properties. The heat-generating composition 1621 in powder or granule form is loaded in heat-generating layer 162, and the thermal-insulation layer 163 is in contact with the skin 17.

In this example, the above three layers of the face mask 10 are adhered together by, for example, using the adhesive or thermal bonding, which are applied to the skin as a whole. In some cases, the thermal-insulation layer is not adhered together with the outer layer and heat-generating layer. That is, in use, the thermal-insulation layer is first applied to the skin, then the outer layer and heat-generating layer are applied, and the similar self-heating thermal-insulation effect can also be obtained.

In this example, the outer layer 161 is made of nonwoven, the upper sealing surface and lower sealing surface of the heat-generating layer 162 are made of air permeable film, and the thermal-insulation layer 163 is a metal-plating film made of medicinal thermal-insulation film and silvery or golden metal. In practice, as long as the outer layer 161 and the upper sealing surface of the heat-generating layer 162 (with or without the lower sealing surface) are made of air permeable material, there is no specific limitation to the air permeable material, all materials which can make air permeate into the heat-generating composition are suitable.

The thermal-insulation layer 163 is a metallized polyethylene terephthalate (MPET) film made of PET film and silvery or golden metal. In this example, the thermal-insulation layer is formed by a physical vapor deposition process, during which the PET film is coated with a layer of metal.

Further, the heat-generating layer 162 in this example is provided with griddings 1622, and the heat-generating composition 1621 in powder or granule form is loaded in each gridding 1622. Due to the griddings 1622, the heat-generating composition 1621 in powder or granule form would not accumulate in a certain area of the thermal-insulation layer 162 under gravity action during use or transit, so that the thermal-insulation layer gives off heat evenly and local high temperature would not occur.

In this example, the griddings 1622 are formed by thermal bonding of the upper sealing surface of heat-generating layer 162 in contact with the outer layer 161 and the lower sealing surface of heat-generating layer 162 in contact with the thermal-insulation layer 163. The griddings 1622 and the upper/lower sealing surface of heat-generating layer 162 are directly molded in one. In production, the bonding mold of thermal-insulation layer is provided with gridding patterns. After heating, the upper/lower sealing surface will be bonded together on the bulge of the bolding mold.

In this example, the self-heating thermal-insulation face mask is loaded in a sealed package before using, so as to prevent the heat-generating composition in heat-generating layer from being oxidized by oxygen and water in the air before using. The sealed package can be made from any air impermeable material, such as plastic film.

In this example, the heat-generating composition comprises (by weigh): 50 parts medical iron powder; 15 parts active carbon; 5 parts metal salt; 3 parts vermiculite; 1 part water-absorbent resin; 5 parts water; and 1 part diatomite.

In this example, the thickness of the face mask depends upon the heat-generating layer, since both the outer layer and thermal-insulation layer are made of films very thin. In order to maintain heating temperature of 38-55°Cand heating time of 10-25 minutes, the mask is 2-8mm thick, that is, the total heat-generating composition loaded in the heat-generating layer is 2-8mm thick. Within the thickness range, the mask fits tightly to the face.

### Example 9

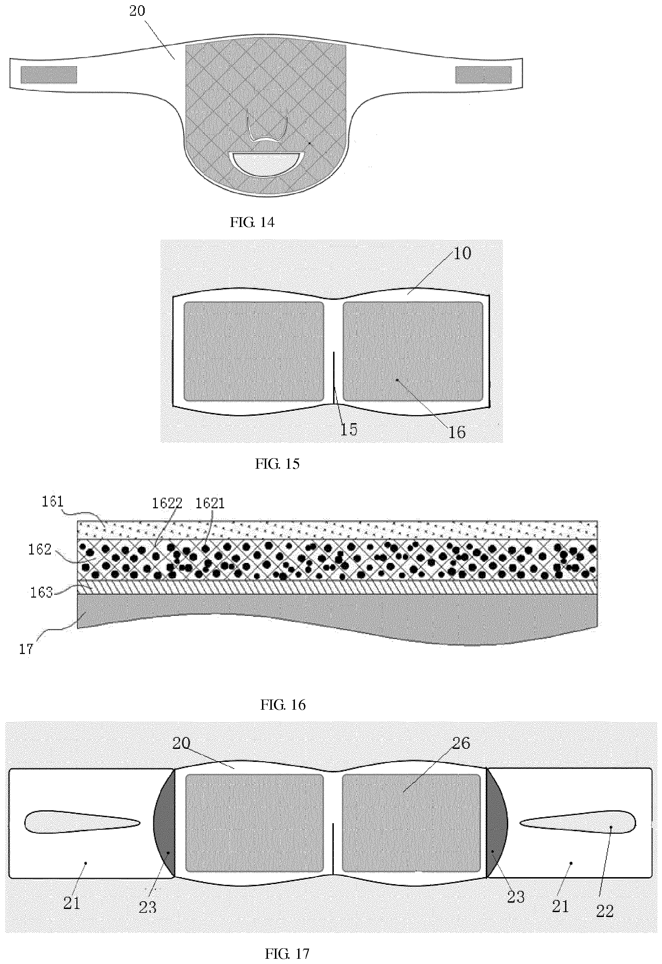

Figure 14 shows another example of the present face mask. The difference of example 9 over example 8 is that there is no notch provided on the portions corresponding to the user's eyes. That is to say, the face mask is inclosed on eye portions.

Since there is no notch on the portions corresponding to the user's eyes, so the face mask can clean and rejuvenate most areas of the face except the mouth and nostril. In addition, the heat-generating layer and thermal-insulation layer of the face mask can warm and nourish the user's eyes, and prevent dry eye syndrome.

### Example 10

In this example, there is provided a layer of water-soluble hydrogel on the surface of the thermal-insulation layer that is in contact with the skin, which is used for adhering the face mask to the skin.

### Example 11

Example 11 differs from example 8 only in the heat-generating composition, which comprises (by weigh): 30 parts medical iron powder; 10 parts active carbon; 1 part metal salt; 13 parts vermiculite; 5 parts water-absorbent resin; 1 part water; and 5 parts diatomite.

### Technical advantage of Examples 8-11

In order to test the skincare and cosmetic effect of the face mask, 50 testees were recruited. All testees first applied a commercial available traditional mask of some brand to the whole face, then applied the three-dimensional face mask in Example 11 on their left face or right face and held the face mask for 15 minutes. After removing the traditional mask and the present face mask, the testees were asked and observed how well the face mask worked. As a result, these testees wildly believed that it was more comfortable on the side of the face applied with the present film, and it was verified by monitoring the changes of moisture content of the face skin that it had higher moisture content on the side of the face applied with both the present face and the traditional mask compared with the other side of the face only applied with the traditional mask. In addition, it was observed that the side of the face applied with both the present face mask and traditional mask appeared more smooth and nutritious.

The present self-heating thermal-insulation face mask is structurally simple, is not constrained by environmental resources, does not require energy supply from an external energy source, is safe and stable, uniformly generates heat, and can effectively prolong heating time and reduce peak heating temperature. The heat given off by the heat-generating layer and maintained by the thermal-insulation layer makes the skin comfortable, improves blood circulation and enlarges facial pores, so as to clean skin dirt and sebaceous secretion. When used in combination with a traditional nourishing face mask or skincare product, the absorption of the effective ingredients in said traditional face mask or skincare product is improved, so the amount of skincare product used is reduced and skincare effect is enhanced. In addition, the use is convenient, environmentally friendly and low-cost. The existing face mask only has single function, without self-heating function, so it is difficult for the skin to absorb the active ingredients (especially in the case that the environment temperature is low and the facial pores are constringed). In addition, during winter months or in cold areas, the cold feeling caused by traditional sheet-mask or mud-mask usually makes the user give up using the mask, so the skin is not nursed when the nutrient is needed the most. The present face mask has overcome the above shortcomings of the existing mask and has expanded the functionality of the existing mask.

Furthermore, the present self-heating thermal-insulation face mask is low cost and disposable, it is safe and hygienic when in contact with face skin or other susceptible parts, and avoids the need for disinfecting the physical heating apparatus which is reused and in direct contract with the skin and potential health risk caused by irregular disinfection. The used face mask will not cause toxin pollution, and the heat-generating composition can be recycled and used as materials improving the quality of the earth.

### Example 12

As shown in Figure 15, in example 12, the eye portion 16 of the eye mask 10 comprises a structure of at least three layers. Figure 16 shows the section view of the structure of three layers, which from outside to inside are respectively: an outer layer 161 formed of air permeable material; a heat-generating layer 162 loaded with heat-generating composition 1621 that generates heat on contact with air, at least an upper sealing surface of the heat-generating layer that is in contact with the outer layer being formed of an air permeable material; and an thermal-insulation layer 163 formed of a material having waterproof and thermal-insulation properties. The heat-generating composition 1621 in powder or granule form is loaded in heat-generating layer 162, and the thermal-insulation layer 163 is in contact with the skin 17.

In this example, the above three layers of the eye mask 10 are adhered together by, for example, using the adhesive or thermal bonding, which are applied to the skin as a whole. In some cases, the thermal-insulation layer is not adhered together with the outer layer and heat-generating layer. That is, in use, the thermal-insulation layer is first applied to the skin, then the outer layer and heat-generating layer are applied, and the similar self-heating thermal-insulation effect can also be obtained.

The eye mask is mainly used for eye care, such as moisturizing, eliminating fatigue, relieving swelling and black eyes, and anti-aging. In this example, the eye portion of the eye mask comprises a structure of three layers, the heat given off from the heat-generating layer can relax the muscle around the eyes, enlarge blood vessel and accelerate blood circulation, so as to relieve swelling, dry eye syndrome and eye irritation. In addition, a thermal-insulation layer formed of a material having waterproof and thermal-insulation properties is provided between the heat-generating layer and skin. When used, the thermal-insulation layer ensures the heat given off from the heat-generating layer is evenly and stably transferred to the skin, and the heat will not spread out quickly from the skin, so as to avoid scalding caused by too high initial temperature and maintain stable and suitable holding temperature. Further, the thermal-insulation layer prevents the water or oil in the skincare product, topically-applied medicine or healthcare product or sweat from permeating into the heat-generating composition, so as to avoid uncontrollable peak heating temperature and cross-contamination between the chemical reaction products of the heat- generating composition and the water or oil in the skincare product, or healthcare product or the sweat, which may cause skin infection. When applied to the face, the heat given off by the heat-generating layer and maintained by the thermal-insulation layer makes the skin comfortable, improves blood circulation and enlarges facial pores, so as to clean skin dirt and sebaceous secretion. When used in combination with a skincare product, the absorption of the effective ingredients in said traditional face mask or skincare product is improved, so the amount of skincare product used is reduced and skincare effect is enhanced. In addition, the use is convenient, environmentally friendly and low-cost.

In this example, the outer layer 161 is made of nonwoven, the upper sealing surface and lower sealing surface of the heat-generating layer 162 are made of air permeable film, and the thermal-insulation layer 163 is a metal-plating film made of medicinal thermal-insulation film and silvery or golden metal. In practice, as long as the outer layer 161 and the upper sealing surface of the heat-generating layer 162 (with or without the lower sealing surface) are made of air permeable material, there is no specific limitation to the air permeable material, all materials which can make air permeate into the heat-generating composition are suitable. The air permeability of the air permeable material can be controlled by adjusting the size and number of the aperture on the air-permeable film, which vary depending upon the heat-generating composition and the amount thereof, and the desired heating temperature. There is no limitation to the thickness of the air permeable material, as long as it has no affection on the desired effect. Thinner air permeable material is better while achieving the air-permeable property and supporting function.

The thermal-insulation layer 163 is a metallized polyethylene terephthalate (MPET) film made of PET film and silvery or golden metal.

Further, the heat-generating layer 162 in this example is provided with griddings 1622, and the heat-generating composition 1621 in powder or granule form is loaded in each gridding 1622. Due to the griddings 1622, the heat-generating composition 1621 in powder or granule form would not accumulate in a certain area of the thermal-insulation layer 162 under gravity action during use or transit, so that the thermal-insulation layer gives off heat evenly and local high temperature would not occur.

In this example, the griddings 1622 are formed by thermal bonding of the upper sealing surface of heat-generating layer 162 in contact with the outer layer 161 and the lower sealing surface of heat-generating layer 162 in contact with the thermal-insulation layer 163. The griddings 1622 and the upper/lower sealing surface of heat-generating layer 162 are directly molded in one. In production, the bonding mold of thermal-insulation layer is provided with gridding patterns. After heating, the upper/lower sealing surface will be bonded together on the bulge of the bolding mold. Of course, the griddings can be formed by any other known method in the prior art.

In this example, the self-heating thermal-insulation eye mask is loaded in a sealed package before using, so as to prevent the heat-generating composition in heat-generating layer from being oxidized by oxygen and water in the air before using. The sealed package can be made from any air impermeable material, such as plastic film.

In this example, the heat-generating composition comprises (by weigh): 50 parts medical iron powder; 15 parts active carbon; 5 parts metal salt; 3 parts vermiculite; 1 part water-absorbent resin; 5 parts water; and 1 part diatomite.

In this example, the thickness of the eye mask depends upon the heat-generating layer, since both the outer layer and thermal-insulation layer are made of films very thin. In order to maintain heating temperature of 38-55°Cand heating time of 10-25 minutes, the mask is 2-8mm thick, that is, the total heat-generating composition loaded in the heat-generating layer is 2-8mm thick. Within the thickness range, the mask fits tightly to the face.

In this example, a slit 15 is vertically cut along a lower half of a central vertical axis of the eye mask. Due to this, after applied to the eyes, a left side piece and a right side piece of the eye mask can naturally splay outward on the portion of nose bridge, so as to fit the user's eyes in shape, that is, the left side piece and the right side piece of the eye mask are symmetrically arranged along the nose bridge and naturally splay outward to the two sides of the slit so as to be reliably adhered to the eyes.

### Example 13

Figure 17 shows another example of the present eye mask. The difference of example 13 over example 12 is that besides the structure of three layers on the eyes portions, the eye mask 20 is also provided with two hanging parts 21. The hanging parts 21 are adhered to the main body of the eye mask, and drawing reference 23 represents the adhering site. The hanging parts 21 have ears slits 22 which can hung on the user's ears. In use, the eye mask can be reliably attached to the user's eyes by the hanging parts 21, so the eye mask would not easily move or fall off from the eyes.

### Example 14

In this example, there is provided a layer of water-soluble hydrogel on the surface of the thermal-insulation layer of the eye mask that is in contact with the skin.

### Example 15

Example 15 differs from example 12 only in the heat-generating composition, which comprises (by weigh): 30 parts medical iron powder; 10 parts active carbon; 1 pars metal salt; 13 parts vermiculite; 5 parts water-absorbent resin; 1 part water; and 5 parts diatomite.

### Technical advantage of Examples 12-15

In order to test the skincare and cosmetic effect of the eye mask, 50 testees were recruited. All testees first applied a commercial available traditional eye mask of some brand, then applied the eye mask in example 15 on their left eye or right eye and held the eye mask for 15 minutes. After removing the traditional eye mask and the present eye mask, the testees were asked and observed how well the eye mask worked. As a result, these testees wildly believed that the eye applied with the present eye mask was more comfortable, and the eye fatigue could be eased to some extent.

The present self-heating thermal-insulation eye mask is structurally simple, is not constrained by environmental resources, does not require energy supply from an external energy source, is safe and stable, uniformly generates heat, and can effectively prolong heating time and reduce peak heating temperature. The heat given off by the heat-generating layer and maintained by the thermal-insulation layer makes the skin comfortable, improves blood circulation and enlarges facial pores, so as to clean skin dirt and sebaceous secretion. When used in combination with a traditional nourishing eye mask or skincare product, the absorption of the effective ingredients in said traditional eye mask or skincare product is improved, so the amount of skincare product used is reduced and skincare effect is enhanced. In addition, the use is convenient, environmentally friendly and low-cost. The existing eye mask only has single function, without self-heating function, so it is difficult for the skin to absorb the active ingredients (especially in the case that the environment temperature is low and the facial pores are constringed). In addition, during winter months or in cold areas, the cold feeling caused by traditional sheet-mask or mud-mask usually makes the user give up using the mask, so the skin is not nursed when the nutrient is needed the most. The present eye mask has overcome the above shortcomings of the existing eye mask and has expanded the functionality of the existing eye mask.

Furthermore, the present self-heating thermal-insulation eye mask is low cost and disposable, it is safe and hygienic when in contact with face skin or other susceptible parts, and avoids the need for disinfecting the physical heating apparatus which is reused and in direct contract with the skin and potential health risk caused by irregular disinfection. The used eye mask will not cause toxin pollution, and the heat-generating composition can be recycled and used as materials improving the quality of the earth.

## Claims

**1.** A self-heating thermal-insulation film, comprising a structure of at least three layers, which from outside to inside are respectively:
an outer layer formed of an air permeable material;
a heat-generating layer loaded with heat-generating composition that generates heat on contact with air, at least an upper sealing surface of the heat-generating layer that is in contact with the outer layer being formed of an air permeable material; and
an thermal-insulation layer formed of a material having waterproof and thermal-insulation properties.

**2.** The self-heating thermal-insulation film according to claim 1, wherein the thermal-insulation layer is a metallized polyethylene terephthalate film made of polyethylene terephthalate film and metal.

**3.** The self-heating thermal-insulation film according to claim 2, wherein the metal used for the metallized polyethylene terephthalate film is aluminum, nickel, chromium or a mixture thereof.

**4.** The self-heating thermal-insulation film according to claim 1, wherein the thermal-insulation layer is provided with griddings, and the heat-generating composition is evenly distributed in the griddings.

**5.** The self-heating thermal-insulation film according to claim 4, wherein the griddings are formed by thermal bonding of the upper sealing surface of the heat-generating layer in contact with the outer layer and a lower sealing surface of heat-generating layer in contact with the thermal-insulation layer.

**6.** The self-heating thermal-insulation film according to claim 1, wherein the outer layer is made of nonwoven or woven fabric of natural or synthetic fiber.

**7.** The self-heating thermal-insulation film according to claim 1, wherein the self-heating thermal-insulation film is loaded in a sealed package before using.

**8.** The self-heating thermal-insulation film according to claim 1, wherein the heat-generating composition in the heat-generating layer comprises an oxidizable metal, active carbon, an inorganic metal salt, water, a polymer moisturizer, and an absorbent.

**9.** The self-heating thermal-insulation film according to any of the preceding claims, wherein the film is ergonomically designed in shape to be applied to various body parts.

**10.** The self-heating thermal-insulation film according to claim 9, wherein there is provided a layer of water-soluble hydrogel on the surface of the thermal-insulation layer that is in contact with the skin, the layer of soluble hydrogel being used for adhering the film to the skin.

**11.** The self-heating thermal-insulation film according to claim 10, wherein there is provided an active ingredient of a topically-applied healthcare product, a topically-applied medicine or a skincare product in the layer of water-soluble hydrogel.

**12.** The self-heating thermal-insulation film according to any of claims 9-11, wherein the self-heating thermal-insulation film is made into a face mask applied to the face.

**13.** The self-heating thermal-insulation film according to claim 12, wherein the heat-generating layer of the face mask maintains a heating temperature at 38°C to 55°C, and the heating time lasts 10 to 25 minutes.

**14.** The self-heating thermal-insulation film according to claim 13, wherein the heat-generating composition in the heat-generating layer comprises 30 to 50 parts by weight medical iron powder, 10 to 15 parts by weight active carbon, 1 to 5 parts by weight a metal salt, 3 to 13 parts by weight vermiculite, 1 to 5 parts by weight a water-absorbent resin, and 1 to 5 parts by weight water.

**15.** The self-heating thermal-insulation film according to claim 13, wherein the heat-generating composition in the heat-generating layer comprises 30 to 50 parts by weight medical iron powder, 10 to 15 parts by weight active carbon, 1 to 5 parts by weight a metal salt, 3 to 13 parts by weight vermiculite, 1 to 5 parts by weight a water-absorbent resin, 1 to 5 parts by weight water, and 1 to 5 parts by weight diatomite.

**16.** The self-heating thermal-insulation film according to claim 14 or 15, wherein the face mask is 2-8mm thick.

**17.** A method of using the self-heating thermal-insulation film according to claim 9, comprising applying a topically-applied medicine or healthcare product to the body prior to applying the film; or,
pre-coating a topically-applied medicine or healthcare product on a surface of the thermal-insulation layer that is in contact with the skin, and then directly applying the pre-coated film to the body.

**18.** A method of using the self-heating thermal-insulation film according to claim 12, comprising applying a skincare product or a traditional nourishing face mark to the face prior to applying the face mask; or, pre-coating a skincare product on the surface of the thermal-insulation layer that is in contact with the skin, and then directly applying the pre-coated face mask to the face.

**19.** A self-heating thermal-insulation three-dimensional face mask which has a facial concave-convex shape formed by joining two sheets together, the face portion of the three-dimensional face mask corresponding to the face comprising a structure of at least three layers, which from outside to inside are respectively: an outer layer formed of an air permeable material; a heat-generating layer loaded with heat-generating composition that generates heat on contact with air, at least an upper sealing surface of the heat-generating layer that is in contact with the outer layer being formed of an air permeable material; and an thermal-insulation layer formed of a material having waterproof and thermal-insulation properties.

**20.** The self-heating thermal-insulation three-dimensional face mask according to claim 19, wherein the thermal-insulation layer is a metallized polyethylene terephthalate film made of polyethylene terephthalate film and golden or silvery metal.

**21.** The self-heating thermal-insulation three-dimensional face mask according to claim 20, wherein the metal used for the metallized polyethylene terephthalate film is aluminum, nickel, or a mixture thereof.

**22.** The self-heating thermal-insulation three-dimensional face mask according to claim 19, wherein the thermal-insulation layer is provided with griddings, and the heat-generating composition is evenly distributed in the griddings.

**23.** The self-heating thermal-insulation three-dimensional face mask according to claim 22, wherein the griddings are formed by thermal bonding of the upper sealing surface of the heat-generating layer in contact with the outer layer and a lower sealing surface of heat-generating layer in contact with the thermal-insulation layer.

**24.** The self-heating thermal-insulation three-dimensional face mask according to claim 19, wherein the outer layer is made of nonwoven or woven fabric of natural or synthetic fiber.

**25.** The self-heating thermal-insulation three-dimensional face mask according to claim 19, wherein the self-heating thermal-insulation three-dimensional face mask is loaded in a sealed package before using.

**26.** The self-heating thermal-insulation three-dimensional face mask according to claim 19, wherein the heat-generating composition in the heat-generating layer comprises an oxidizable metal, active carbon, an inorganic metal salt, water, a polymer moisturizer, and an absorbent.

**27.** The self-heating thermal-insulation three-dimensional face mask according to claim 26, wherein the heat-generating layer of the face mask maintains a heating temperature at 38-55°C, and the heating time lasts 10-25 minutes.

**28.** The self-heating thermal-insulation three-dimensional face mask according to claim 27, wherein the heat-generating composition in the heat-generating layer comprises 30 to 50 parts by weight medical iron powder, 10 to 15 parts by weight active carbon, 1 to 5 parts by weight a metal salt, 3 to 13 parts by weight vermiculite, 1 to 5 parts by weight a water-absorbent resin, and 1 to 5 parts by weight water.

**29.** The self-heating thermal-insulation three-dimensional face mask according to claim 27, wherein the heat-generating composition in the heat-generating layer comprises 30 to 50 parts by weight medical iron powder, 10 to 15 parts by weight active carbon, 1 to 5 parts by weight a metal salt, 3 to 13 parts by weight vermiculite, 1 to 5 parts by weight a water-absorbent resin, 1 to 5 parts by weight water, and 1 to 5 parts by weight diatomite.

**30.** The self-heating thermal-insulation three-dimensional face mask according to claim 27 or 28, wherein the face mask is 2-8mm thick.

**31.** The self-heating thermal-insulation three-dimensional face mask according to claim 19, wherein there is provided a layer of water-soluble hydrogel on the surface of the thermal-insulation layer that is in contact with the skin, the layer of soluble hydrogel being used for adhering the face mask to the skin.

**32.** The self-heating thermal-insulation three-dimensional face mask according to claim 31, wherein there is provided an active ingredient of a topically-applied healthcare product, a topically-applied medicine or a skincare product in the layer of water-soluble hydrogel.

**33.** The self-heating thermal-insulation three-dimensional face mask according to claim 19, wherein the three-dimensional face mask comprises a left side sheet and a right side sheet respectively matching the left area of the face and the right area of the face, front edge portions of the left side sheet and the right side sheet are provided with an adhesive material, the left side sheet and the right side sheet are thereby adhered together by the adhesive material along a median line of the face to form a facial concave-convex shape.

**34.** The self-heating thermal-insulation three-dimensional face mask according to claim 33, wherein either of the front edge portions of the left side sheet and the right side sheet has a protrusion projecting outwards from above down and matching the shape of the left area or right area of the external nose, a mouth portion which is a notch matching the mouth is provided below the protrusion, a portion of the front edge portion below the notch has an arc shape with a certain radian and extends downwards to the bottom the face mask; except the notch matching the mouth, either of the front edge portions of the left side sheet and the right side sheet is provided with an adhesive material, and the left side sheet and the right side sheet are thereby adhered together by the adhesive material.

**35.** The self-heating thermal-insulation three-dimensional face mask according to claim 34, wherein a glabella portion of either of the front edge portions of the left side sheet and the right side sheet which faces a space between the user's eyebrows, just above the protrusion, is formed as an arc which is slightly concaved inwards.

**36.** The self-heating thermal-insulation three-dimensional face mask according to claim 34, wherein an eye-and-glabella portion of either of the front edge portions of the left side sheet and the right side sheet which faces the user's eyes and glabella, just above the protrusion, is formed as a notch which is concaved inwards; except the eye-and-glabella portion and the mouth portion, either of the front edge portions of the left side sheet and the right side sheet are provided with an adhesive material , and the left side sheet and the right side sheet are thereby adhered together by the adhesive material.

**37.** The self-heating thermal-insulation three-dimensional face mask according to any of claims 33-36, there is a fixing strap provided on either of the rear edge portions of the left side sheet and the right side sheet, and there is a velcro provided at the end of the fixing strap.

**38.** A method of using the self-heating thermal-insulation three-dimensional face mask according to any of claims 33-36, comprising first applying a skincare product or a traditional nourishing face mark to the face prior to applying the three-dimensional face mask; or, pre-coating a skincare product on the surface of the thermal-insulation layer that is in contact with the skin, joining the left side sheet and the right side sheet together, then applying the joined three-dimensional face mask to the face.

**39.** A self-heating thermal-insulation face mask, the face portion of the face mask corresponding to the face comprising a structure of at least three layers, which from outside to inside are respectively: an outer layer formed of air permeable material; a heat-generating layer loaded with heat-generating composition that generates heat on contact with air, at least an upper sealing surface of the heat-generating layer that is in contact with the outer layer being formed of an air permeable material; and an thermal-insulation layer formed of a material having waterproof and thermal-insulation properties.

**40.** The self-heating thermal-insulation face mask according to claim 39, wherein the thermal-insulation layer is a metallized polyethylene terephthalate film made of polyethylene terephthalate film and golden or silvery metal.

**41.** The self-heating thermal-insulation face mask according to claim 40, wherein the metal used for the metallized polyethylene terephthalate film is aluminum, nickel, or a mixture thereof.

**42.** The self-heating thermal-insulation face mask according to claim 39, wherein the thermal-insulation layer is provided with griddings, and the heat-generating composition is evenly distributed in the griddings.

**43.** The self-heating thermal-insulation face mask according to claim 42, wherein the griddings are formed by thermal bonding of the upper sealing surface of heat-generating layer in contact with the outer layer and the lower sealing surface of heat-generating layer in contact with the thermal-insulation layer.

**44.** The self-heating thermal-insulation face mask according to claim 39, wherein the outer layer is made of nonwoven or woven fabric of natural or synthetic fiber.

**45.** The self-heating thermal-insulation face mask according to claim 39, wherein the self-heating thermal-insulation face mask is loaded in a sealed package before using.

**46.** The self-heating thermal-insulation face mask according to claim 39, wherein the heat-generating composition in the heat-generating layer comprises an oxidizable metal, active carbon, an inorganic metal salt, water, a polymer moisturizer, and an absorbent.

**47.** The self-heating thermal-insulation face mask according to claim 46, wherein the heat-generating layer of the face mask maintains a heating temperature at 38-55°C , and the heating time lasts 10-25 minutes.

**48.** The self-heating thermal-insulation face mask according to claim 47, wherein the heat-generating composition in the heat-generating layer comprises 30 to 50 parts by weight medical iron powder, 10 to 15 parts by weight active carbon, 1 to 5 parts by weight a metal salt, 3 to 13 parts by weight vermiculite, 1 to 5 parts by weight a water-absorbent resin, and 1 to 5 parts by weight water.

**49.** The self-heating thermal-insulation face mask according to claim 47, wherein the heat-generating composition in the heat-generating layer comprises 30 to 50 parts by weight medical iron powder, 10 to 15 parts by weight active carbon, 1 to 5 parts by weight a metal salt, 3 to 13 parts by weight vermiculite, 1 to 5 parts by weight a water-absorbent resin, 1 to 5 parts by weight water, and 1 to 5 parts by weight diatomite.

**50.** The self-heating thermal-insulation face mask according to claim 47 or 48, wherein the face mask is 2-8mm thick.

**51.** The self-heating thermal-insulation face mask according to claim 39, wherein there is provided a layer of water-soluble hydrogel on the surface of the thermal-insulation layer that is in contact with the skin, the layer of soluble hydrogel being used for adhering the face mask to the user's body.

**52.** The self-heating thermal-insulation face mask according to claim 51, wherein there is provided an active ingredient of a topically-applied healthcare product, a topically-applied medicine or a skincare product in the layer of water-soluble hydrogel.

**53.** The self-heating thermal-insulation face mask according to claim 39, wherein the face mask has notches matching the user's nose and mouth, respectively.

**54.** The self-heating thermal-insulation face mask according to claim 53, wherein the face mask also has notches matching the user's eyes.

**54.** The self-heating thermal-insulation face mask according to claim 53 or 54, wherein there is a fixing strap provided on either side of the face mask, and there is a velcro provided at the end of the fixing strap.

**56.** A method of using the self-heating thermal-insulation face mask according to any of claims 53-55, comprising: applying a skincare product or a traditional nourishing face mark to the face prior to applying the self-heating thermal-insulation face mask; or, pre-coating a skincare product on the surface of the thermal-insulation layer that is in contact with the skin, then applying the pre-coated self-heating thermal-insulation face mask to the face.

**57.** A self-heating thermal-insulation eye mask, the eye portion of the eye mask corresponding to the user's eyes comprising a structure of at least three layers, which from outside to inside are respectively: an outer layer formed of air permeable material; a heat-generating layer loaded with heat-generating composition that generates heat on contact with air, at least an upper sealing surface of the heat-generating layer that is in contact with the outer layer being formed of air permeable material; and an thermal-insulation layer formed of a material having waterproof and thermal- insulation properties.

**58.** The self-heating thermal-insulation eye mask according to claim 57, wherein the thermal-insulation layer is a metallized polyethylene terephthalate film made of polyethylene terephthalate film and golden or silvery metal.

**59.** The self-heating thermal-insulation eye mask according to claim 58, wherein the metal used for the metallized polyethylene terephthalate film is aluminum, nickel, or a mixture thereof.

**60.** The self-heating thermal-insulation eye mask according to claim 57, wherein the thermal-insulation layer is provided with griddings, and the heat-generating composition is evenly distributed in the griddings.

**61.** The self-heating thermal-insulation eye mask according to claim 60, wherein the griddings are formed by thermal bonding of the upper sealing surface of heat-generating layer in contact with the outer layer and the lower sealing surface of heat-generating layer in contact with the thermal-insulation layer.

**62.** The self-heating thermal-insulation eye mask according to claim 57, wherein the outer layer is made of nonwoven or woven fabric of natural or synthetic fiber.

**63.** The self-heating thermal-insulation eye mask according to claim 57, wherein the self-heating thermal-insulation eye mask is loaded in a sealed package before using.

**64.** The self-heating thermal-insulation eye mask according to claim 57, wherein the heat-generating composition in the heat-generating layer contains an oxidizable metal, active carbon, an inorganic metal salt, water, a polymer moisturizer, and an absorbent.

**65.** The self-heating thermal-insulation eye mask according to claim 64, wherein the heating temperature of the heat-generating layer of the eye mask is maintained at 38-55°C , and the heating time lasts 10-25 minutes.

**66.** The self-heating thermal-insulation eye mask according to claim 65, wherein the heat-generating composition in the heat-generating layer comprises 30 to 50 parts by weight medical iron powder, 10 to 15 parts by weight active carbon, 1 to 5 parts by weight a metal salt, 3 to 13 parts by weight vermiculite, 1 to 5 parts by weight a water-absorbent resin, and 1 to 5 parts by weight water.

**67.** The self-heating thermal-insulation eye mask according to claim 65, wherein the heat-generating composition in the heat-generating layer comprises 30 to 50 parts by weight medical iron powder, 10 to 15 parts by weight active carbon, 1 to 5 parts by weight a metal salt, 3 to 13 parts by weight vermiculite, 1 to 5 parts by weight a water-absorbent resin, 1 to 5 parts by weight water, and 1 to 5 parts by weight diatomite.

**68.** The self-heating thermal-insulation eye mask according to claim 65 or 66, wherein the eye mask is 2-8mm thick.

**69.** The self-heating thermal-insulation eye mask according to claim 57, wherein there is provided a layer of water-soluble hydrogel on a surface of the thermal-insulation layer that is in contact with the skin, the layer of soluble hydrogel being used for adhering the eye mask to the skin.

**70.** The self-heating thermal-insulation eye mask according to claim 69, wherein there is provided an active ingredient of a topically-applied healthcare product, a topically-applied medicine or a skincare product in the layer of water-soluble hydrogel.

**71.** The self-heating thermal-insulation eye mask according to claim 57, wherein the eye mask comprises hanging parts which have ears slits and can be hung on the user's ears.

**72.** The self-heating thermal-insulation eye mask according to claim 57 or 71, wherein a slit is vertically cut along a lower half of a central vertical axis of the eye mask.
